# EUROPÄISCHE PATENTSCHRIFT

(11) **EP 0 772 578 B1**
(45) Veröffentlichungstag und Bekanntmachung des Hinweises auf die Patenterteilung: **19.09.2001**
(21) Anmeldenummer: 95925847.6
(22) Anmeldetag: 08.07.1995
(51) Int. Cl.: C07C 45/00, C07C 49/17, C07C 49/84, C07C 49/83, C07C 49/493, C07D 307/52, C07D 213/50, C07C 47/56, C07C 69/67

(54) **VERFAHREN ZUR HERSTELLUNG VON ACYLOINEN**
METHOD OF PRODUCING ACYLOINES
PROCEDE DE FABRICATION D'ACYLO NES

(30) Priorität: 19.07.1994 DE 4425436
(43) Veröffentlichungstag der Anmeldung: 14.05.1997
(73) Patentinhaber: BASF AKTIENGESELLSCHAFT, 67056 Ludwigshafen (DE)
(72) Erfinder: EBEL, Klaus, D-68623 Lampertheim (DE); SCHNEIDER, Regina, D-67136 Fussgönheim (DE); MELDER, Johann-Peter, D-68165 Mannheim (DE); TELES, Joaquim, Henrique, D-67059 Ludwigshafen (DE)
(86) Internationale Anmeldenummer: EP9502660
(87) Internationale Veröffentlichungsnummer: WO9602484

(56) Entgegenhaltungen:
- EP-A- 0 219 317
- EP-A- 0 587 044
- FR-A- 2 280 618

## Beschreibung

Die vorliegende Erfindung betrifft ein Verfahren zur Herstellung von Acyloinen der allgemeinen Formel I 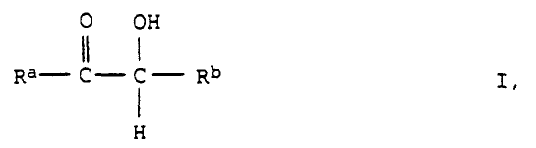 in der R^{a} und R^{b} gleich oder verschieden sind und für Wasserstoff oder eine gegebenenfalls substituierte C₁- bis C₂₀-Alkyl-, eine gegebenenfalls substituierte C₆- bis C₁₀-Aryl-, eine gegebenenfalls substituierte C₇- bis C₁₂-Aralkyl-, eine gegebenenfalls substituierte Heteroaryl- oder eine gegebenenfalls substituierte Heterocycloalkylgruppe stehen, ausgenommen Autokondensationsprodukte des Formaldehyds.

Des weiteren betrifft die vorliegende Erfindung ein Verfahren zur Herstellung cycloaliphatischer oder heterocycloaliphatischer Acyloine mit insgesamt 5 bis 12 Ringgliedern.

Außerdem betrifft die vorliegende Erfindung ein Verfahren zur Herstellung von Acyloinen der allgemeinen Formel Ia 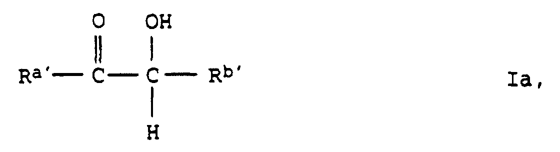 in der die Reste R^{a'} und R^{b'} verschieden sind und für Wasserstoff oder eine gegebenenfalls substituierte C₁- bis C₂₀-Alkyl-, eine gegebenenfalls substituierte C₆- bis C₁₀-Aryl-, eine gegebenenfalls substituierte C₇- bis C₁₁-Aralkyl-, eine gegebenenfalls substituierte Heteroaryl- oder eine gegebenenfalls substituierte Heterocycloalkylgruppe stehen, ausgenommen Autokondensationsprodukte des Formaldehyds.

Seit den Arbeiten von Breslow (J. Am. Chem. Soc. 80, 3719 (1959)) ist bekannt, daß man Thiazoliumylide als Katalysatoren für die katalytische Umpolung von Aldehyden und somit für deren Kondensation zu Acyloinen verwenden kann. Castells (Tetrahedron Lett. 21, 4517 (1980)) wandte diese Reaktion auf die Selbstkondensation von Formaldehyd an. Als Acyloine werden im allgemeinen α-Hydroxycarbonylverbindungen bezeichnet, insbesondere α-Hydroxyketone und α-Hydroxyaldehyde. Die Verfahren haben allerdings erhebliche Nachteile. So werden relativ große Mengen, nämlich 5 bis 20 Mol-%, bezogen auf den eingesetzten Aldehyd, des als Katalysator dienenden Thiazoliumylids benötigt, um eine zufriedenstellende Raum-Zeit-Ausbeute zu erzielen. Außerdem erweisen sich die Thiazoliumylid-Katalysatoren im Dauerbetrieb als relativ instabil, d.h. es bilden sich in erheblichem Maße Abbauprodukte des Thiazoliumylid-Katalysators, die sich vom gewünschten Produkt praktisch nicht abtrennen lassen. Diese Sachverhalte stehen einer Anwendung der Thiazoliumylid-Katalysatoren in technischen Verfahren entgegen.

DE-A 42 30 466 betrifft ein Verfahren zur Herstellung von Autokondensationsprodukten des Formaldehyds mittels Triazoliumsalzkatalysatoren. Der in diesem Verfahren als Ausgangsmaterial eingesetzte Formaldehyd ist als einfachster Aldehyd im Hinblick auf sein chemisches Verhalten, insbesondere seine hohe chemische Reaktivität, einzigartig unter den Aldehyden. Gleiches gilt, aufgrund von deren besonderer chemischer Struktur, für die intermediär in diesem verfahren gebildeten Formaldehyd-Triazoliumsalz-Addukte. Darüber hinaus gibt es bei der Kondensation von Formaldehyd mit sich selbst praktisch keine sterische Hinderung der Reaktionspartner. Ein Hinweis auf die Anwendbarkeit dieses Verfahrens auf die Kondensation höherer Aldehyde als Formaldehyd wird in dieser Schrift nicht gegeben.

Acyloine sind aufgrund ihrer Bifunktionalität - Carbonyl- und Hydroxygruppe in α-Stellung zueinander - sehr gut zur Synthese von Heterocyclen, insbesondere Imidazolen (EP-A 252 162) und Imidazolonen (J. Chem. Soc. Perkin II, 310 (1981)) geeignet, die wiederum z.B. bei der Herstellung von Arznei- und Pflanzenschutzmitteln Verwendung finden und sind somit gesuchte Zwischenprodukte und Synthesebausteine zur Herstellung solcher Wirkstoffe. Infolge ihres hohen Reduktionsvermögens werden Acyloine als Reduktionsmittel in der Färberei, beispielsweise zum Färben von textilen Materialien aus Cellulosefasern (EP-A 364 752) verwendet. weitere Anwendungen, von Acyloinen sind beispielhaft im Folgenden angeführt:
Acetoin wird z.B. als Aromastoff in Lebensmitteln verwendet, ebenso das daraus durch Oxidation erhältliche Diacetyl. Furoin dient als Ausgangsmaterial zur Herstellung von Furildioxim, das daraus durch dessen Oxidation zu Furil und dessen anschließende Oximierung mit Hydroxylamin erhalten und als Reagenz zur analytischen Bestimmung von Schwermetallkationen, beispielsweise Nickelionen, verwendet wird. Benzoin ist beispielsweise das unmittelbare vorprodukt bei der Herstellung von Benzil, das als Virusstatikum pharmazeutische Verwendung findet und weiterhin als Ausgangsstoff zur Herstellung von Antimykotika und Konservierungsmitteln dient.

Der vorliegenden Erfindung lag somit die Aufgabe zugrunde, ein Verfahren zur Herstellung von Acyloinen aus den entsprechenden, gut zugänglichen Aldehyden zu finden, das nicht mit den Nachteilen der Thiazoliumylid-katalysierten Acyloinherstellung behaftet ist. Ferner sollte das Verfahren in der Lage sein, sogenannte gekreuzte Acyloine, das sind Acyloine, die durch Kondensation zweier verschiedener Aldehyde entstehen, auf wirtschaftliche Weise zugänglich zu machen.

Dementsprechend wurde ein Verfahren zur Herstellung von Acyloinen der allgemeinen Formel I 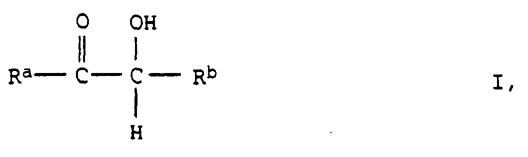 in der R^{a} und R^{b} gleich oder verschieden sind und für Wasserstoff oder eine gegebenenfalls substituierte C₁- bis C₂₀-Alkyl-, eine gegebenenfalls substituierte C₆- bis C₁₀-Aryl-, eine gegebenenfalls substituierte C₇- bis C₁₂-Aralkyl-, eine gegebenenfalls substituierte Heteroaryl- oder eine gegebenenfalls substituierte Heterocycloalkylgruppe stehen, ausgenommen Autokondensationsprodukte des Formaldehyds gefunden, das dadurch gekennzeichnet ist, daß man einen Aldehyd der Formel II

R^{a}CHO II

mit einem Aldehyd der Formel III

R^{b}CHO III,

in denen R^{a} und R^{b} die obengenannte Bedeutung haben und wenigstens einer der Reste R^{a} und R^{b} einen anderen Rest als Wasserstoff bedeutet, in Gegenwart von Katalysatoren umsetzt, die aus Triazoliumsalzen der Formel IV 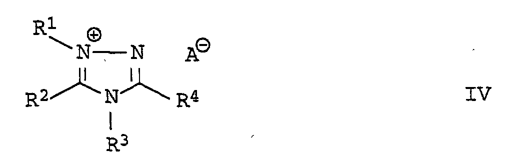 in der
R¹ und R³ gleich oder verschieden sind und für aliphatische Gruppen mit 1 bis 30 Kohlenstoffatomen, C₃- bis C₂₀-Cycloalkyloder -alkenylgruppen, C₃- bis C₂₀-Heterocycloalkyl- oder -alkenylgruppen, über ein Kohlenstoffatom an den Triazoliumring gebundene C₂- bis C₃₀-Alkoxygruppen mit einem oder mehreren Sauerstoffatomen in der Etherkette, C₁- bis C₃₀-Fluor, Chlor- und/oder Brom enthaltende Halogenalkylgruppen mit einem oder mehreren Halogenatomen, über ein Kohlenstoffatom an den Triazoliumring gebundene C₂- bis C₃₀-sekundäre Aminogruppen oder C₃- bis C₃₀-tertiäre Aminogruppen für gegebenenfalls substituierte Arylgruppen, für gegebenenfalls substituierte Aralkylgruppen und/oder für gegebenenfalls substituierte Heteroarylgruppen stehen,
R² Wasserstoff oder die Gruppe R^{b}CH(OH) ist und in der
R⁴ den Resten R¹ und R³ gleich oder verschieden ist oder Wasserstoff, ein Halogenatom, eine Nitro- oder Cyanogruppe, eine aliphatische Gruppe mit 1 bis 30 Kohlenstoffatomen, C₃- bis C₂₀-Cycloalkyl- oder -alkenylgruppen, C₃- bis C₂₀-Heterocycloalkyl- oder -alkenylgruppen, über ein Kohlenstoffatom an den Triazoliumring gebundene C₂- bis C₃₀-Alkoxygruppen mit einem oder mehreren Sauerstoffatomen in der Etherkette, C₁- bis C₃₀-Fluor, Chlor- und/oder Brom enthaltende Halogenalkylgruppen mit einem oder mehreren Halogenatomen, über ein Kohlenstoffatom an den Triazoliumring gebundene C₂- bis C₃₀-sekundäre Aminogruppen oder C₃- bis C₃₀-tertiäre Aminogruppen, eine gegebenenfalls substituierte Arylgruppe, eine gegebenenfalls substituierte Aralkylgruppe, eine gegebenenfalls substituierte Heteroarylgruppe, eine über das Sauerstoffatom an den Triazoliumring gebundene Alkoxygruppe -OR⁵, eine über das Schwefelatom an den Triazoliumring gebundene Thioethergruppe -SR⁶, eine über das Stickstoffatom an den Triazoliumring gebundene Aminogruppe -NR⁷R⁸, eine Acylgruppe COR⁹ oder eine Estergruppe -COOR¹⁰ bedeutet, wobei die Reste R⁵, R⁶, R⁷, R⁸ und R⁹ für Reste stehen, wie sie für R¹ genannt wurden und R¹⁰ eine C₁- bis C₃₀-Alkyl- oder eine gegebenenfalls substituierte Aryl- oder Aralkylgruppe ist,
   oder in der der Rest R³ gemeinsam mit dem Rest R⁴ eine C₃- bis C₅-Alkylen- oder Alkenylen- oder eine C₆- bis C₁₄-Arylen-, eine C₇- bis C₁₄-Aralkylen- oder C₈- bis C₁₄-Aralkenylen-Brücke bildet, und

A das Äquivalent eines ein- oder mehrfach negativ geladenen Anions zur elektrischen Neutralisierung der Ladung des Triazoliumkations ist,
mit Hilfe einer Hilfsbase erzeugt worden sind.

Weiterhin wurde ein Verfahren zur Herstellung cycloaliphatischer oder heterocycloaliphatischer Acyloine mit insgesamt 5 bis 12 Ringgliedern gefunden, das dadurch gekennzeichnet ist, daß man einen gegebenenfalls substituierten aliphatischen C₅- bis C₁₂-Dialdehyd oder einen heteroaliphatischen Dialdehyd mit einer Kettenlänge von 4 bis 11 Kohlenstoffatomen, der zusätzlich in der Kette eine O , S oder N(R^{c}) Gruppe enthält, in der R^{c} eine C₁- bis C₄-Alkyl- oder Acylgruppe ist, mit einem Katalysator gemäß den Ansprüchen 1 bis 6 umsetzt.

Außerdem wurde ein Verfahren zur Herstellung von gekreuzten Acyloinen der allgemeinen Formel Ia 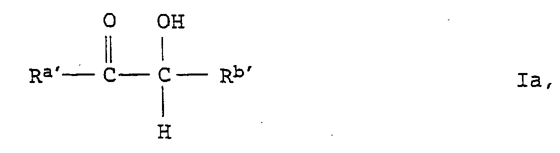 in der die Reste R^{a'} und R^{b'} verschieden sind und für Wasserstoff oder eine gegebenenfalls substituierte C₁- bis C₂₀-Alkyl-, eine gegebenenfalls substituierte C₆- bis C₁₀-Aryl-, eine gegebenenfalls substituierte C₇- bis C₁₂-Aralkyl-, eine gegebenenfalls substituierte Heteroaryl- oder eine gegebenenfalls substituierte Heterocycloalkylgruppe stehen, ausgenommen Dihydroxyaceton, Glycerinaldehyd sowie C₄- und C₅-Zucker gefunden, das dadurch gekennzeichnet ist, daß man einen Aldehyd der Formel II

R^{a}CHO II

mit einem Triazoliumsalz der Formel IVa 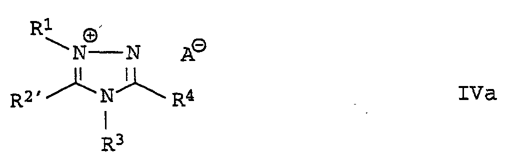 in der
R¹ und R³ gleich oder verschieden sind und für aliphatische Gruppen mit 1 bis 30 Kohlenstoffatomen, C₃- bis C₂₀-Cycloalkyloder -alkenylgruppen, C₃- bis C₂₀-Heterocycloalkyl- oder -alkenylgruppen, über ein Kohlenstoffatom an den Triazoliumring gebundene C₂- bis C₃₀-Alkoxygruppen mit einem oder mehreren Sauerstoffatomen in der Etherkette, C₁- bis C₃₀-Fluor, Chlor- und/oder Brom enthaltende Halogenalkylgruppen mit einem oder mehreren Halogenatomen, über ein Kohlenstoffatom an den Triazoliumring gebundene C₂- bis C₃₀-sekundäre Aminogruppen oder C₃- bis C₃₀-tertiäre Aminogruppen für gegebenenfalls substituierte Arylgruppen, für gegebenenfalls substituierte Aralkylgruppen und/oder für gegebenenfalls substituierte Heteroarylgruppen stehen,
R^{2'} die Gruppe R^{b}CH(OH) darstellt, und in der
R⁴ den Resten R¹ oder R³ gleich oder verschieden ist oder Wasserstoff, ein Halogenatom, eine Nitro- oder Cyanogruppe, eine aliphatische Gruppe mit 1 bis 30 Kohlenstoffatomen, C₃- bis C₂₀-Cycloalkyl- oder -alkenylgruppen, C₃- bis C₂₀-Heterocycloalkyl- oder -alkenylgruppen, über ein Kohlenstoffatom an den Triazoliumring gebundene C₂- bis C₃₀-Alkoxygruppen mit einem oder mehreren Sauerstoffatomen in der Etherkette, C₁- bis C₃₀-Fluor, Chlor- und/oder Brom enthaltende Halogenalkylgruppen mit einem oder mehreren Halogenatomen, über ein Kohlenstoffatom an den Triazoliumring gebundene C₂- bis C₃₀-sekundäre Aminogruppen oder C₃- bis C₃₀-tertiäre Aminogruppen, eine gegebenenfalls substituierte Arylgruppe, eine gegebenenfalls substituierte Aralkylgruppe, eine gegebenenfalls substituierte Heteroarylgruppe, eine über das Sauerstoffatom an den Triazoliumring gebundene Alkoxygruppe -OR⁵, eine über das Schwefelatom an den Triazoliumring gebundene Thioethergruppe -SR⁶, eine über das Stickstoffatom an den Triazoliumring gebundene Aminogruppe -NR⁷R⁸, eine Acylgruppe -COR⁹ oder eine Estergruppe -COOR¹⁰ bedeutet, wobei die Reste R⁵, R⁶, R⁷, R⁸ und R⁹ für Reste stehen, wie sie für R¹ genannt wurden und R¹⁰ eine C₁- bis C₃₀-Alkyl- oder eine gegebenenfalls substituierte Aryl- oder Aralkylgruppe ist,
   oder in der der Rest R³ gemeinsam mit dem Rest R⁴ eine C₃- bis C₅-Alkylen- oder Alkenylen- oder eine C₆- bis C₁₄-Arylen-, eine C₇- bis C₁₄-Aralkylen- oder C₈- bis C₁₄-Aralkenylen-Brücke bildet, und

A das Äquivalent eines ein- oder mehrfach negativ geladenen Anions zur elektrischen Neutralisierung der Ladung des Triazoliumkations ist,
in Gegenwart einer Hilfsbase und in einem Molverhältnis Triazoliumsalz IVa/Aldehyd II von 1:1 bis 5:1 umsetzt, mit der Maßgabe, daß mindestens einer der Reste R^{a} oder R^{b} von Wasserstoff verschieden ist.

Das erfindungsgemäße Verfahren betrifft somit die Herstellung acyclischer oder cyclischer Acyloine, mit Ausnahme der Herstellung von Autokondensationsprodukten des Formaldehyds, wie sie bei der Umsetzung von Formaldehyd mit Katalysatoren der Formel IV oder V entstehen, also beispielsweise Glykolaldehyd, Glycerinaldehyd, Dihydroxyaceton sowie C₄- und C₅-Zuckern. Insofern das erfindungsgemäße Verfahren die Herstellung acyclischer Acyloine betrifft, können damit sowohl sogenannte einheitliche Acyloine durch die Kondensation zweier Aldehyde R^{a}CHO II und R^{b}CHO III, in denen die Reste R^{a} und R^{b} gleich, jedoch von Wasserstoff verschieden sind, als auch sogenannte gekreuzte Acyloine durch die Kondensation zweier verschiedener Aldehyde R^{a}CHO II und R^{b}CHO III hergestellt werden.

Bei den erfindungsgemäß zu verwendenden Katalysatoren handelt es sich somit um Katalysatoren, die aus 1,2,4-Triazoliumsalzen IV mit Hilfe einer Hilfsbase erzeugt werden. Da die Reste R¹, R³ und R⁴ in der Regel lediglich einen Einfluß auf das Löslichkeitsverhalten der Triazoliumsalze haben, können diese Reste eine Vielzahl von Bedeutungen haben.

So können R¹, R³ und R⁴ gleich oder verschieden sein und für aliphatische Gruppen mit 1 bis 30 Kohlenstoffatomen, wie C₁- bis C₃₀-Alkyl-, vorzugsweise C₁- bis C₁₀-Alkylgruppen, C₂- bis C₃₀-, vorzugsweise C₂- bis C₁₀-Alkenyl- oder Alkinylgruppen mit 1 oder 2, vorzugsweise nur mit einer Mehrfachbindung, C₃- bis C₂₀-, vorzugsweise C₃- bis C₁₀-Cycloalkyl- oder -alkenyl-Gruppen, C₃- bis C₂₀-Heterocycloalkyl- oder -alkenyl-Gruppen, wie Piperidinyl-, Piperazinyl-, Pyrrolidinyl-, Imidazolidinyl-, Tetrahydrothienyl-, Tetrahydrofuranyl-, Tetrahydropyranyl-, Thiazolidinyl-, Oxazolidinyl-, Imidazolinyl-, Thiazolinyl-, Oxazolinyl- oder Kronenether-Gruppen, über ein Kohlenstoffatom an den Triazoliumoder Tetrazoliumring gebundene C₂- bis C₃₀-, vorzugsweise C₂- bis C₁₀-Alkoxygruppen mit einem oder mehreren, vorzugsweise nur mit einem Sauerstoffatom in der Etherkette, C₁- bis C₃₀-, vorzugsweise C₁- bis C₁₀-Fluor-, Chlor- und/oder Brom-, vorzugsweise Fluorund/oder Chlor-, insbesondere Fluor-enthaltende Halogenalkylgruppen mit einem oder mehreren, vorzugsweise mit 1 bis 3 Halogenatomen, im Falle von Fluoralkylgruppen vorteilhaft auch perfluorierte Fluoralkylgruppen, über ein Kohlenstoffatom an den Triazoliumring gebundene Aminogruppen, wie C₂- bis C₃₀-, vorzugsweise C₂- bis C₁₀-sekundäre Aminogruppen, C₃- bis C₃₀-, vorzugsweise C₃- bis C₂₁-tertiäre Aminogruppen, für gegebenenfalls substituierte Arylgruppen, vorzugsweise C₆- bis C₁₄-Arylgruppen, insbesondere für Phenyl-, Naphthyl-, Anthryl- oder PhenanthrylGruppen, für gegebenenfalls substituierte C₇- bis C₂₀-Aralkylgruppen, insbesondere die Benzyl-, Phenylethylen- oder Naphthylmethylen-Gruppe, oder für gegebenenfalls substituierte C₂- bis C₁₅-Heteroarylgruppen mit 1 bis 3 Stickstoffatomen oder einem Sauerstoff- oder Schwefelatom oder mit 1 bis 2 Stickstoffatomen oder einem Sauerstoff- oder Schwefelatom im Ring, wie die Furanyl-, Thienyl-, Pyrrolyl-, Pyrazolyl-, Imidazolyl-, Oxazolyl-, Thiazolyl-, Isothiazolyl-, Isoxazolyl-, Pyridinyl-, Pyrimidinyl-, Pyrazinyl-, Chinolinyl-, Naphthyridinyl-, 1,2,4-Triazolyl- oder Acridinyl-Gruppen, stehen.

Sowohl die aliphatischen als auch die aromatischen und selbstverstandlich auch die Aralkylreste können einfach oder mehrfach, vorzugsweise nicht mehr als dreifach, mit Halogenatomen, Nitro-, Hydroxy-, Cyano-, Alkyl-, Alkoxy- oder Aminogruppen substituiert sein. Da diese Substituenten in der Regel eine nur geringfügige Wirkung auf die katalytische Aktivität der aus IV bzw. V erzeugten Katalysatoren ausüben, werden, hauptsächlich aufgrund der kostengünstigeren Herstellung, die obengenannten, unsubstituierten Reste R¹, R³ und R⁴ bevorzugt verwendet.

Außer den obengenannten Bedeutungen kann der Rest R⁴ im Unterschied zu den Resten R¹ und R³ ein Wasserstoff, eine Nitrooder Cyanogruppe, ein Halogenatom, ausgewählt aus Fluor, Chlor oder Brom, eine über das Sauerstoffatom an den Triazoliumring gebundene Alkoxygruppe -OR⁵, eine über das Schwefelatom an den Triazoliumring gebundene Thioethergruppe -SR⁶, eine über das Stickstoffatom an den Triazoliumring gebundene Aminogruppe -NR⁷R⁸, eine Acylgruppe -COR⁹ oder eine Estergruppe- COOR¹⁰ sein. Die Reste R⁵, R⁶, R⁷, R⁸ und R⁹ können die gleichen Bedeutungen haben, wie sie oben für den Rest R¹ angegeben sind. Ebenso wie die Reste R¹ können auch die Reste R⁵, R⁶, R⁷, R⁸, R⁹ und R¹⁰ die für die Reste R¹ genannten Substituenten tragen, vorzugsweise, aufgrund der kostengünstigeren Herstellung, werden in der Regel aber unsubstituierte Reste R⁵, R⁶, R⁷, R⁸, R⁹ und R¹⁰ gewählt. Bevorzugte Reste R⁵, R⁶, R⁷, R⁸, R⁹ und R¹⁰ sind C₁- bis C₃₀-, insbesondere C₁- bis C₁₀-Alkylgruppen, C₆- bis C₁₀-Arylgruppen, insbesondere die Phenyl- oder Naphthylgruppe sowie C₇- bis C₂₀-, vorzugsweise C₇- bis C₁₄-Aralkylgruppen, insbesondere die Benzyl-, Phenylethylen- oder Naphthylmethylen-Gruppe. Ein weiterer bevorzugter Rest R⁷ ist die Hydroxymethylengruppe. Weitere bevorzugte Reste R⁷ sind solche, die in α-Stellung zum Stickstoffatom eine Hydroxygruppe tragen.

Der Rest R⁴ kann weiterhin gemeinsam mit dem Rest R³ eine C₃- bis C₅-Alkylen- oder Alkenylenbrücke-, eine C₆- bis C₁₄-Arylenbrücke, vorzugsweise eine o-Phenylen-, o-Naphthylen-, 1,8-Naphthylen-, o-Fluorenylen-, 5,4-Fluorenylen-, o-Phenanthrylen-, 5,4-Phenanthrylen-, 9,10-Phenanthrylen-, o-Anthrylen-, 1,9-Anthrylenoder eine 2,2'-Biphenylenbrücke, eine C₇- bis C₁₄-Aralkyl- oder Aralkenylenbrücke bilden, wobei diese verbrückenden Reste R³∩R⁴ einfach oder mehrfach, vorzugsweise nicht mehr als 3fach mit Halogenatomen, Nitro-, Hydroxy-, Cyano-, Alkyl-, Alkoxy- oder Aminogruppen substituiert sein können. Da diese Substituenten in der Regel eine nur geringfügige Wirkung auf die katalytische Aktivität der betreffenden aus IV erzeugten Katalysatoren ausüben, werden in der Regel, hauptsächlich auf Grund ihrer kostengünstigeren Herstellung, unsubstituierte verbrückende Reste R³∩R⁴ bevorzugt. Da sowohl der Rest R³ als auch der Rest R⁴ die Heteroatome Stickstoff, Sauerstoff oder Schwefel enthalten können, kann auch der verbrückende Rest R³∩R⁴ diese Heteroatome enthalten, vorzugsweise enthält der verbrückende Rest R³∩R⁴ nicht mehr als 2, insbesondere nicht mehr als eines dieser Heteroatome.

Der Rest R², der sich am mutmaßlich katalytisch aktiven Zentrum der Triazoliumverbindungen IV befindet, kann im Falle der Ausgestaltungen des erfindungsgemäßen Verfahrens zur Herstellung der Acyloine der Formel I Wasserstoff oder die Gruppe R^{b}CH(OH) sein, vorzugsweise ist der Rest R² Wasserstoff. Im Falle der gezielten Herstellung gekreuzter Acyloine der Formel Ia ist der Rest R² vorzugsweise die Gruppe R^{b}CH(OH). Bei der Herstellung alicyclischer oder heterocycloaliphatischer Acyloine aus den entsprechenden Dialdehyden ist der Rest R² vorzugsweise Wasserstoff.

Die Anionen, die das Anion-Äquivalent A zur elektrischen Neutralisierung der Ladung des Triazolium-Kations bilden, können im Prinzip beliebig gewählt werden, vorzugsweise werden aber die nicht-nukleophilen Anionen von Mineralsäuren oder starken Carbonsäuren gewählt. Diese Anionen können ein- oder mehrfach, bevorzugt nicht mehr als dreifach negativ geladen sein. Derartige mehrfach negativ geladene Anionen können die ihrer negativen Ladung entsprechende Anzahl Triazolium-Kationen elektrisch neutralisieren und elektrostatisch, also salzartig, an sich binden. Das Anion-Aquivalent A entspricht somit der zur elektrischen Neutralisation einer molaren Menge des Triazolium-Kations benötigten molaren Menge eines einfach oder mehrfach negativ geladenen Anions dividiert durch dessen Ladungszahl.

Geeignete Anionen sind beispielsweise die Anionen der Halogenide, wie Fluorid, Chlorid, Bromid oder Iodid, Nitrat, Tetrafluoroborat, Tetraphenylborat, Hexafluorophosphat, Hexachloroplatinat, Perchlorat, Sulfat, Phosphat, Trifluoracetat, Methansulfonat oder Toluolsulfonat. Ebenso können saure Kationenaustauscher in ihrer anionischen Form, beispielsweise Polyacrylate, sulfonierte Phenol-Formaldehyd-Harze oder sulfoniertes Polystyrol, als Polyanionen wirken. Vorzugsweise werden die Halogenide, Nitrat, Tetrafluoroborat sowie Perchlorat als Anion verwendet.

Die Wirkungsweise der erfindungsgemäß eingesetzten Triazoliumsalze IV ist noch weitgehend unbekannt und es können lediglich Mutmaßungen über den dem erfindungsgemäßen Verfahren zugrunde liegenden chemischen Mechanismus aufgestellt werden. Die Ergebnisse aller bisherigen Experimente deuten jedoch darauf hin, daß das Triazoliumsalz IV durch die Hilfsbase in 5-Position des Triazoliumringes deprotoniert wird, wobei das mit dem Carben-VII mesomere Ylid-VI gebildet wird (siehe Gleichung (1)), 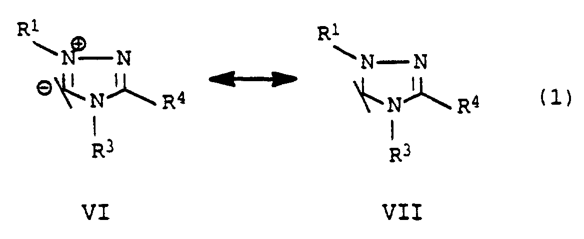 von dem vermutet wird, daß es die eigentliche katalytisch aktive Spezies ist. Es ist zwar möglich, daß unter den Reaktionsbedingungen auch noch andere katalytisch aktive Spezies dieser Triazoliumverbindungen vorliegen, die sich von dem Ylid-VI/ Carben-VII ableiten lassen und mit diesen im Gleichgewicht stehen, Voraussetzung für die Entwicklung einer katalytischen Aktivität, scheint jedoch das Durchlaufen der reaktiven Zwischenstufe des Triazolium-Ylids/Carbens-VI/VII zu sein, auf welche Weise und ausgehend von welchen Ausgangsmaterialien dieses auch immer generiert werden mag. Diese Darlegungen sind jedoch lediglich als Versuch einer Erklärung der erfindungsgemäßen Umsetzung zu betrachten. Sollte zukünftig einmal festgestellt werden, daß andere reaktive Zwischenstufen als die hier postulierten, die erfindungsgemäße Umsetzung katalysieren, so ist dies für den Schutzumfang der vorliegenden Anmeldung als unerheblich zu betrachten, da diese Spezies schließlich durch die erfindungsgemäßen Maßnahmen erzeugt werden.

Einen Hinweis darauf, daß Position 5 des Triazoliumringes höchstwahrscheinlich das katalytisch aktive Zentrum dieser Katalysatoren ist, gibt der Umstand, daß bei der Umsetzung von Alkoholaten, beispielsweise Methanolaten, oder Thiolaten mit den Triazoliumsalzen IV Alkoholat- bzw. Thiolat-Additionsprodukte der Formel V 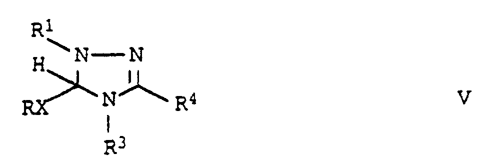 isoliert werden können, in der X für Sauerstoff oder Schwefel steht, R eine C₁- bis C₄-Alkylgruppe, vorzugsweise die Methylgruppe ist und in der R¹, R³ und R⁴ die obengenannte Bedeutung haben. Setzt man diese Additionsverbindungen V an Stelle der Triazoliumsalze IV mit den Aldehyden II und III unter den üblicherweise im erfindungsgemäßen Verfahren angewandten Reaktionsbedingungen, jedoch in Abwesenheit der Hilfsbase, um, so bilden sich ebenfalls die Acyloine I. Wahrscheinlich wird aus den Verbindungen V unter den üblicherweise angewandten Reaktionsbedingungen der Alkohol bzw. das Thiol RXH wieder abgespalten und das mutmaßlich katalytisch aktive Ylid-VI bzw. das Carben-VII erzeugt, welches dann seine katalytische Aktivität entfalten kann.

Des weiteren ist es möglich, die Additionsverbindungen V allein, in fester Form oder in einem hochsiedenden Lösungsmittel zu erhitzen, dabei den betreffenden Alkohol bzw. das betreffende Thiol abzuspalten und auf diese Weise Verbindungen zu erzeugen, die besonders aktive Katalysatoren für die Kondensation der Aldehyde II und III sind. Höchstwahrscheinlich entsteht bei dieser Thermolyse das entsprechende Ylid-VI bzw. das Carben-VII oder eine diesen Spezies äquivalent wirkende Verbindung.

Somit ergeben sich für das erfindungsgemäße Verfahren zur Herstellung von Acyloinen I dreierlei Ausgestaltungen:
- α): Verwendung der Triazoliumsalzverbindungen IV als Katalysatoren in Gegenwart einer Hilfsbase.
- β): Verwendung der Additionsverbindungen V als Katalysatoren.
- γ): Verwendung der durch die thermische Eliminierung der Alkohole ROH bzw. Thiole RSH aus den Additionsverbindungen V erhaltenen Thermolyseprodukte als Katalysatoren.

Allen diesen drei Ausgestaltungen des erfindungsgemäßen Verfahrens ist gemäß den vorausgegangenen Darlegungen gemein, daß die Kondensation der Aldehyde II oder III letztendlich von der gleichen katalytisch aktiven Spezies katalysiert wird. Alle diese drei Ausgestaltungen sind daher einander äquivalent, obgleich diese drei Verfahrensvarianten unterschiedliche Vorteile haben, die im Einzelfall den Ausschlag für die Anwendung der einen oder der anderen Variante geben:

Variante α) hat den Vorteil, daß die Triazoliumverbindungen IV nicht weiter derivatisiert werden müssen. Die Varianten β) und γ) haben den Vorteil, daß sie in Abwesenheit einer Hilfsbase durchgeführt werden können. Da die Anwesenheit einer Hilfsbase im Reaktionsgemisch gegebenenfalls zu unerwünschten Nebenreaktionen der Acyloine Anlaß geben kann, wiegt dieser Vorteil im Einzelfall besonders schwer. Die gemäß Variante γ) eingesetzten katalytisch aktiven Verbindungen sind besonders aktive Katalysatoren. Besonders geeignet zur Durchführung der Variante β) des erfindungsgemäßen Verfahrens sind die Methanolat-Additionsverbindungen Va.

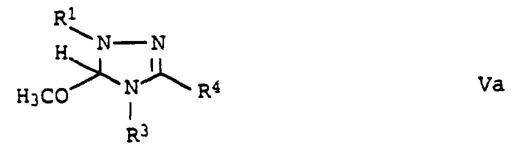

Im erfindungsgemäßen Verfahren können als Ausgangsaldehyde R^{a}CHO II bzw. R^{b}CHO III prinzipiell beliebige Aldehyde eingesetzt werden. Die Aldehyde R^{a}CHO II und R^{b}CHO III, die erfindungsgemäß miteinander zu den entsprechenden Acyloinen umgesetzt werden, können gleich oder verschieden sein, wobei die Selbstkondensation von Formaldehyd nicht zum erfindungsgemäßen Verfahren zählt.

Als Ausgangsaldehyde II und III können beispielsweise aliphatische, cycloaliphatische, aromatische, heteroaromatische, heterocycloaliphatische und araliphatische Aldehyde verwendet werden.

Obgleich prinzipiell alle aliphatischen Aldehyde im erfindungsgemäßen Verfahren eingesetzt werden können, wird dieses im allgemeinen mit solchen Aldehyden R^{a}CHO II und R^{b}CHO III durchgeführt, in denen die Reste R^{a} und R^{b} für C₁- bis C₂₀-, vorzugsweise C₁- bis C₁₅- und besonders bevorzugt für C₁- bis C₁₀-Alkylgruppen stehen. Diese Alkylgruppen können geradkettig oder verzweigt sein. Vorzugsweise werden geradkettige Aldehyde verwendet. Von den Aldehyden I und II mit verzweigten Alkylketten sind solche mit geringer sterischer Hinderung bevorzugt. Die Alkylgruppen der aliphatischen Aldehyde können unsubstituiert sein oder 1 bis 3 gleiche oder verschiedene, vorzugsweise einen, unter den Reaktionsbedingungen inerten Substituenten tragen. Solche Substituenten sind beispielsweise Halogenatome, wie Fluor-, Chlor-, Brom- oder Iodatome, vorzugsweise Fluor-, Chlor- oder Bromatome, die Nitrilgruppe, die Nitrogruppe, die Oxogruppe, die Hydroxygruppe, die Thiolgruppe, C₁- bis C₂₀-, vorzugsweise C₁- bis C₁₀-, insbesondere C₁- bis C₄-Alkoxygruppen, C₆- bis C₁₀-Aryloxygruppen, insbesondere die Phenoxygruppe, C₇- bis C₁₂-Aralkyloxygruppen, insbesondere die Benzyloxygruppe, cyclische oder acyclische Acetalgruppen, -COOR-Gruppen, in denen R ein aromatischer, aliphatischer oder araliphatischer Rest ist, 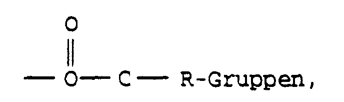 in denen R ein aromatischer, aliphatischer oder araliphatischer Rest ist, Di-(C₁- bis C₁₀-alkyl-), vorzugsweise Di-(C₁- bis C₄-alkyl-)aminogruppen, C₁- bis C₁₀-, vorzugsweise C₁- bis C₄-Alkylthiogruppen, Carboxylat- oder Sulfonat-Gruppen. Weiterhin können die Alkylgruppen C-C-Doppel- oder Dreifachbindungen enthalten.

Die Reste R^{a} und R^{b} können auch cycloaliphatische Gruppen sein, beispielsweise C₃- bis C₈-, vorzugsweise C₃- bis C₆-Cycloalkylgruppen, oder heterocycloaliphatische Gruppen mit insgesamt vorzugsweise 5 bis 8, insbesondere 5 bis 6 Ringgliedern, wobei diese heterocycloaliphatischen Gruppen, je nach deren Ringgröße, 1 bis 3, vorzugsweise 1 bis 2, insbesondere eine, Oxa-, Thia- oder (N-R)-diyl-Gruppen, worin R eine Alkyl-, Aryl-, Aralkyl- oder Acyl-Gruppe ist, enthalten können. Die cycloaliphatischen oder heterocycloaliphatischen Reste R^{a} und R^{b} können unsubstituiert sein oder 1 bis 3 gleiche oder verschiedene, vorzugsweise einen, Substituenten tragen. Beispielsweise können die cycloaliphatischen oder heterocycloaliphatischen Reste R^{a} und R^{b} mit solchen Substituenten substituiert sein, wie sie oben für die aliphatischen Reste R^{a} und R^{b} genannt wurden. Das Substitutionsmuster der cycloaliphatischen oder heterocycloaliphatischen Reste R^{a} und R^{b} ist für die Durchführbarkeit des erfindungsgemäßen Verfahrens in der Regel nicht kritisch.

Bevorzugte Ausgangsverbindungen R^{a}CHO II und R^{b}CHO III sind weiterhin solche, in denen die Reste R^{a} und R^{b} für C₆- bis C₁₀-Arylgruppen, beispielsweise die Phenyl- oder Naphthylgruppe stehen. Diese Arylgruppen können unsubstituiert sein oder 1 bis 3 gleiche oder verschiedene, vorzugsweise einen, Substituenten tragen. Als Substituenten der Arylgruppen kommen beispielsweise solche Substituenten in Frage, wie sie oben für die aliphatischen Reste R^{a} und R^{b} genannt wurden. Außer mit den am angegebenen Ort genannten Substituenten können die aromatischen Reste R^{a} und R^{b} selbstverständlich auch mit C₁- bis C₁₀-, vorzugsweise C₁- bis C₄-Alkylgruppen substituiert sein. Das Substitutionsmuster der Arylgruppen ist für die Durchführbarkeit des erfindungsgemäßen Verfahrens in der Regel nicht kritisch.

Weiterhin können Aralkyl-aldehyde als Ausgangsverbindungen R^{a}CHO II und R^{b}CHO III im erfindungsgemäßen Verfahren eingesetzt werden, vorzugsweise solche, in denen R^{a} und R^{b} für C₇- bis C₁₂-Aralkylgruppen, insbesondere die Benzylgruppe, stehen. Die C₇- bis C₁₂-Aralkylgruppen können unsubstituiert sein oder 1 bis 3 gleiche oder verschiedene Substituenten tragen. Da der Substitutionsgrad und das Substitutionsmuster dieser Aralkylverbindungen im allgemeinen für die Ausführbarkeit des erfindungsgemäßen Verfahrens nicht kritisch sind, können diese Aralkylreste beispielsweise mit solchen Substituenten substituiert sein, wie sie im vorhergehenden Absatz für die Arylreste R^{a} und R^{b} genannt wurden, in der dort angegebenen Weise substituiert sein.

Als weiteres bevorzugtes Ausgangsmaterial dienen im erfindungsgemäßen Verfahren heteroaromatische Aldehyde R^{a}CHO II und R^{b}CHO III. Die Art der heteroaromatischen Reste R^{a} und R^{b} ist für die Durchführbarkeit des erfindungsgemäßen Verfahrens in der Regel nicht kritisch. Es können sowohl elektronenreiche heteroaromatische Aldehyde, wie Pyrrol-carbaldehyd oder Furancarbaldehyd (Furfurol), als auch elektronenarme heteroaromatische Aldehyde, wie Pyridincarbaldehyde, im erfindungsgemäßen Verfahren eingesetzt werden. Beispielhaft für aromatische Reste R^{a} und R^{b} seien die Furyl-, Pyrrolyl-, Thienyl-, Imidazolyl-, Thiazolyl-, Pyrazolyl-, Oxazolyl-, Triazolyl-, Pyrydyl-, Chinolinyl-, Isochinolyl-, Pyrimidinyl-, Pyrazinyl-, Pyridazinyl-, Indolinyl-, Indazolyl-, Phthalazinyl-, Naphthyridyl-, Chinoxalinyl-, Cinnolinyl-, Purinyl-, Pteridinyl-, Carbazolyl- oder AcridinylGruppen genannt. Diese aromatischen Reste können unsubstituiert sein oder, je nach Art des aromatischen Restes, 1 bis 3, vorzugsweise einen, gleiche oder verschiedene der Substituenten tragen, wie sie oben für die aromatischen Reste R^{a} und R^{b} genannt worden sind.

Die als Ausgangsmaterial zur Herstellung der Acyloine der Formel I dienenden Aldehyde II

R^{a}CHO II

und III

R^{b}CHO III

können gleich oder verschieden sein. Werden Aldehyde mit identischen Resten R^{a} und R^{b} eingesetzt, entsteht als Produkt der Umsetzung nur ein Acyloinprodukt der Formel I. Werden im erfindungsgemäßen Verfahren Aldehyde II und III mit unterschiedlichen Resten R^{a} und R^{b} miteinander umgesetzt, so können bis zu vier verschiedene Reaktionsprodukte erhalten werden. Dies sei beispielhaft für die erfindungsgemäße Umsetzung von Furfurol mit Benzaldehyd erläutert. Hierbei werden neben Benzoin der Formel VIII 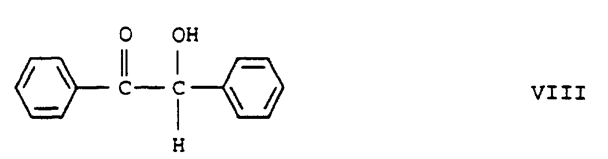 und Furoin der Formel IX 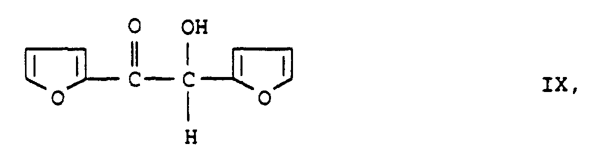 welche jeweils durch die Selbstkondensation von zwei Molekülen Benzaldehyd bzw. Furfurol entstehen, zusätzlich die isomeren, gekreuzten Acyloine der Formeln X 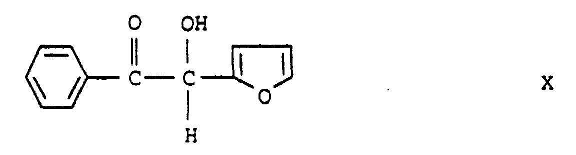 und XI 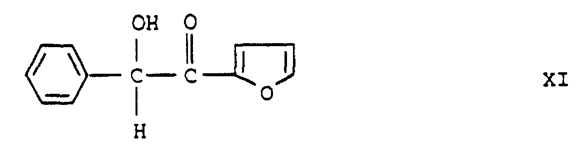 gebildet, die durch die gekreuzte Kondensation von Furfurol und Benzaldehyd entstehen. Analoges gilt im allgemeinen für die Umsetzung anderer Aldehyde II und III mit unterschiedlichen Resten R^{a} und R^{b}.

Die unterschiedlichen Produkte, die bei der Umsetzung zweier verschiedener Aldehyde II und III im erfindungsgemäßen Verfahren entstehen, können auf an sich herkömmliche Weise, beispielsweise durch Kristallisation, Destillation oder mittels chromatographischer Methoden, wie präparativer Gas- oder Flüssigchromatographie, voneinander getrennt werden. Die Mengen, in dem diese unterschiedlichen Reaktionsprodukte im Verhältnis zueinander bei der gekreuzten Kondensation entstehen, ist im allgemeinen von der Art und Reaktivität der eingesetzten Aldehyde II und III abhängig.

Zum Zwecke der praktisch ausschließlichen Herstellung eines bestimmten gekreuzten Acyloins Ia wird vorteilhaft zunächst der Aldehyd R^{b}CHO mit dem Triazoliumsalzkatalysator IV in Abwesenheit einer Hilfsbase inkubiert, wobei sich Triazoliumsalz-AldehydAddukte IVa bilden, in denen R^{2'} die Gruppe R^{b}CH(OH) ist. Diese Addukte werden vorteilhaft in der 1- bis 5-fachen, vorzugsweise in der 1- bis 2-fachen, besonders bevorzugt in einfach stöchiometrischer Menge mit dem betreffenden Aldehyd R^{a}CHO in Gegenwart einer Hilfsbase umgesetzt. Je nach Art der jeweils verwendeten, verschiedenen Aldehyde R^{b}CHO und R^{a}CHO können auf diese Weise die verschiedensten gekreuzten Acyloine mit hoher Selektivität erzeugt werden. Die Inkubation des Triazoliumsalzes IV mit dem Aldehyd R^{b}CHO kann unter den gleichen Reaktionsbedingungen - wie Temperatur, Lösungsmittel etc. - vorgenommen werden, wie die Acyloinkondensation selbst, die nach der Inkubation durch Zugabe der Hilfsbase gestartet wird. Für die Bildung von IVa sind in der Regel wenige Minuten Inkubationszeit ausreichend, die Inkubationszeit kann aber auch praktisch unbegrenzt verlängert werden.

Es wurde erfindungsgemäß gefunden, daß mit Hilfe des erfindungsgemäßen Verfahrens auch alicyclische oder heteroalicyclische Acyloine durch Umsetzung von aliphatischen oder heteroaliphatischen Dialdehyden mit den erfindungsgemäßen Triazoliumkatalysatoren erzeugt werden können.

Zur Herstellung alicyclischer Acyloine dienen vorzugsweise aliphatische α,ω-Dialdehyde mit insgesamt 5 bis 12, vorzugsweise 5 bis 7 Kohlenstoffatomen, beispielsweise α,ω-Pentandial (Glutardialdehyd), α,ω-Hexandial, α,ω-Heptandial, α,ω-Decandial oder α,ω-Dodecandial. Die Dialdehyde können verzweigt sein, vorzugsweise werden unverzweigte Dialdehyde verwendet. Die Alkylenkette der Dialdehyde kann auch Substituenten tragen, wie sie beispielsweise für die aliphatischen Reste R^{a} und R^{b} genannt sind, solange diese die intramolekulare Acyloinkondensation dieser Dialdehyde zu cyclischen Acyloinen nicht sterisch hindern, Bevorzugte Substituenten sind z.B. C₁- bis C₄-Alkylgruppen, Halogenatome, wie Fluor-, Chlor-, Brom- oder Iodatome, vorzugsweise Fluor-, Chloroder Bromatome, die Hydroxylgruppe, die Nitrogruppe, die Nitrilgruppe oder C₁- bis C₄-Alkoxygruppen. Die Anzahl dieser Substituenten kann 1 oder 2, insbesondere 1 sein, vorzugsweise werden unsubstituierte Dialdehyde verwendet. Bei der erfindungsgemäß durchgeführten Acyloinkondensation bilden sich aus diesen Dialdehyden cyclische Acyloine, mit einer Anzahl von Ringgliedern, die der Anzahl der Kohlenstoffatome im eingesetzten Dialdehyd entspricht.

Beispielsweise wird bei der Umsetzung von Glutardialdehyd der Formel XII

OHC-CH₂-CH₂-CH₂-CHO XII

mit den erfindungsgemäßen Katalysatoren 2-Hydroxycyclopentanon der Formel XIII 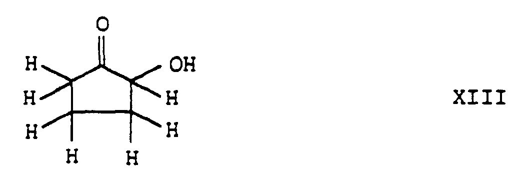 erhalten, d.h. die zweite Aldehydgruppe im Dialdehyd-Molekül addiert sich unter der Einwirkung der erfindungsgemäßen Katalysatoren intramolekular an die erste Aldehydfunktion des Dialdehyds, also analog zu der intermolekularen Kondensation eines Aldehyds R^{a}CHO II mit einem Aldehyd R^{b}CHO III.

Zur Herstellung alicyclischer Acyloine können auch Dialdehyde eingesetzt werden, in denen eine der Methylengruppe des Dialdehyds durch eine O , S oder N(R^{c}) -Gruppe ersetzt ist, in der R^{c} für eine C₁- bis C₄-Alkyl- oder Acylgruppe steht, so daß der verwendete Dialdehyd 4 bis 11, vorzugsweise 5 bis 6 Kohlenstoffatome, zusätzlich zu einer der vorstehend genannten Heterogruppierungen haben kann. Aus diesen Dialdehyden werden im erfindungsgemäßen Verfahren heterocycloaliphatische Acyloine gebildet, deren Anzahl an Ringgliedern der Anzahl an Kohlenstoffatomen plus den in der Alkylenkette befindlichen Heteroatomen entsprechen. Beispielsweise bildet sich aus 3-Oxa-glutardialdehyd der Formel XIV

OHC-CH₂-O-CH₂-CHO XIV

das heterocycloaliphatische Acyloin der Formel XV.

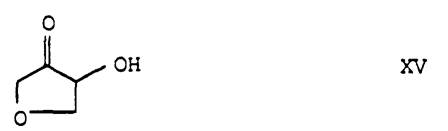

Die Aldehyde R^{a}CHO II und R^{b}CHO können sowohl als solche oder in Form von unter den Reaktionsbedingungen des erfindungsgemäßen Verfahrens Aldehyd-liefernden Verbindungen, also Verbindungen, die unter den Reaktionsbedingungen mit dem freien Aldehyd im Gleichgewicht stehen, eingesetzt werden. Als solche Aldehyd-liefernden Verbindungen sind z.B. die Halbacetale der Aldehyde II und III mit aliphatischen oder aromatischen Alkoholen, vorzugsweise primären C₁- bis C₂₀-, insbesondere C₁- bis C₄-Alkoholen, zu nennen. Ist einer der Aldehyde R^{a}CHO oder R^{b}CHO Formaldehyd, können Formaldehyd-liefernde Verbindungen, wie Paraformaldehyd anstelle von Formaldehyd verwendet werden. Auch die Dialdehyd-Ausgangsverbindungen zur Herstellung cyclischer Acyloine können in Form ihrer Halbacetale eingesetzt werden.

Das erfindungsgemäße Verfahren wird in An- oder Abwesenheit eines Lösungsmittels durchgeführt werden. Als Lösungsmittel eignet sich grundsätzlich ein sehr breites Spektrum von Lösungsmitteln, wie Alkohole, z.B. Methanol, Ethanol, Propanol, Cyclohexanol, 2-Ethylhexanol und Hexadecylalkohol, Amide, z.B. Dimethylformamid (DMF), Dibutylformamid, Harnstoffe, wie Dimethylethylenharnstoff, Dimethylpropylenharnstoff, Carbonate, z.B. Propylencarbonat, Ethylencarbonat, aromatische Lösungsmittel, z.B. Toluol oder Xylol, Heterocyclen, z.B. Pyridin, N-Methylimidazol, N-Methylpyrrolidon, Ketone, z.B. Aceton, Ester, z.B. Essigsäureethylester, Ether, z.B. Methyl-tert.-Butylether, Diethylenglykoldimethylether, Dimethoxyethan, Tetrahydrofuran, Dioxan, aromatische Nitroverbindungen, z.B. Nitrobenzol, Nitrotoluol, tertiäre Amine, z.B. Triethylamin, halogenierte Kohlenwasserstoffe wie Chloroform, Dichlormethan, Chlorbenzol oder Dichlorbenzol, Sulfoxide, wie Dimethylsulfoxid, Sulfone, wie Dimethylsulfon oder Sulfolan, als auch Nitrile, z.B. Acetonitril oder Propionitril.

Die Menge des eingesetzten Lösungsmittels ist im allgemeinen nicht kritisch und von der Art des verwendeten Lösungsmittels abhängig, weshalb zweckmäßigerweise in einem Vorversuch die optimal einzusetzende Lösungsmittelmenge für das betreffende Lösungsmittel ermittelt wird. Als Hilfsbasen zur Aktivierung der Triazoliumsalze IV können eine Vielzahl von Basen eingesetzt werden, die auf Grund ihrer Basenstärke in der Lage sind, die Triazoliumsalze IV in 5-Position oder die Addukte der Triazoliumsalze IV mit dem Aldehyd R^{b}CHO, also die Triazoliumsalze IVa, zu deprotonieren. Vorzugsweise werden dabei nicht-nukleophile Basen verwendet, beispielsweise tertiäre Amine mit 3 bis 30 Kohlenstoffatomen oder tertiäre cyclische Amine, insbesondere auch cyclische Amidine.

Geeignete tertiäre Amine sind beispielsweise Trimethylamin, Triethylamin, Tripropylamin, Tributylamin, Ethyldiisopropylamin, Decyldiethylamin, Tridecylamin, Chinuclidin, Diazabicyclo-[2,2,2]-Octan, N-Methylpiperidin, N-Ethylpiperidin, N-Propylpiperidin, N-Butylpiperidin, N,N'-Dimethylpiperazin, N-Methylmorpholin, Dimethylbenzylamin, Dibenzylmethylamin, Benzyldioctylamin, Benzyldiethylamin, Cyclohexyldiethylamin, Dicylcohexyldiethylamin, Dicyclohexylmethylamin, Dicyclohexylmethylamin, Dicyclohexylethylamin usw. Besonders bevorzugt wird Triethylamin verwendet. Von den cyclischen Amidinen werden 1,5-Diazabicyclo-[4,3,0]-non-5-en, 1,8-Diazabicyclo-[5,4,0]-undec-7-en und 1,5,7-Triazabicyclo-[4,4,0]-dec-7-en vorzugsweise eingesetzt.

Es können auch polymere tertiäre Amine als Hilfsbasen verwendet werden, beispielsweise vernetzte Styrol-Divinylbenzol-Harze oder Phenol-Formaldehyd-Harze, die Seitenketten mit tertiären Aminogruppen tragen oder deren Arylgruppen mit Dialkylaminogruppen substituiert sind. Solche polymeren Amine werden üblicherweise als Anionentauscher eingesetzt.

Außerdem können auch aromatische Stickstoffbasen, wie Chinolin, Pyridin oder N-Alkylimidazole, insbesondere N-C₁- bis C₄-Alkylimidazole zur Deprotonierung der Triazoliumsalze I verwendet werden. Des weiteren können anorganische Basen, wie Alkalimetallund Erdalkalimetall-Hydrogencarbonate, wie Alkalimetall- und Erdalkalimetallcarbonate oder Alkalimetallcarboxylate, insbesondere die Natrium- und Kaliumsalze der C₁- bis C₄-Carbonsäuren, benutzt werden.

Mit Hilfe der Verwendung polymerer tertiäre Amine in Form von Anionenaustauschern ist es möglich, aus den Triazoliumsalzen IV Lösungen der Ylide-VI/Carbene-VII herzustellen, die keine Hilfsbase enthalten. Dazu ist es lediglich erforderlich, die Triazoliumsalze IV an einem tertiäre Aminogruppen tragenden Anionenaustauscherharz zu deprotonieren, beispielsweise indem man eine Lösung des Triazoliumsalzes IV über ein solches Anionenaustauscherharz leitet, und die Aldehyde II und III der Lösung des so erzeugten Ylids-VI/Carbens-VII erst zusetzt, nachdem diese den Anionenaustauscher passiert hat. Diese Arbeitsweise hat den Vorteil, daß durch die Hilfsbase katalysierte Nebenreaktionen, z.B. Aldolkondensationen, weitgehend unterdrückt werden.

Werden als Katalysatoren die Alkoholat- oder Thiolat-Addukte der allgemeinen Formel V oder die aus diesen Verbindungen durch thermische Spaltung erzeugten Ylide-VI/Carbene-VII verwendet, so kann die erfindungsgemäße Umsetzung in Abwesenheit einer Hilfsbase durchgeführt werden. Die Alkoholat- oder Thiolat-Addukte V werden aber aus den entsprechenden Triazoliumsalzen IV durch deren Umsetzung mit dem entsprechenden Alkohol oder Thiol in Gegenwart einer Base erzeugt.

Im erfindungsgemäßen Verfahren können die Aldehyde II und III in Form des freien Aldehyds oder in Form von Aldehyd-liefernden Verbindungen, z.B. in Form ihrer Oligomere, als Aldehydhalbacetale mit C₁- bis C₂₀-, vorzugsweise C₁- bis C₁₀-, insbesondere C₁- bis C₄-Alkoholen, eingesetzt werden. Die Aldehyde können gasförmig oder als Lösung der Reaktionsmischung zugeführt werden. Die Aldehyde werden im allgemeinen bei Temperaturen im Bereich von 0 bis 200°C, bevorzugt bei 10 bis 160°C und besonders bevorzugt bei 20 bis 150°C zusammen mit dem Triazoliumsalz-Katalysator und gegebenenfalls einem organischen Lösungsmittel umgesetzt. Wird der Katalysator gemäß Verfahrensvariante β) aus den Additionsverbindungen der Formel V in situ im Reaktionsgemisch erzeugt, werden im allgemeinen Temperaturen von 20 bis 180°C, vorzugsweise von 60 bis 150°C angewandt. Gelangen hingegen die nach Verfahrensvariante γ) in einer separaten Thermolyse erzeugten, katalytisch aktiven Triazoliumverbindungen zum Einsatz, kann bei Temperaturen von 0 bis 160°C, vorzugsweise von 20 bis 120°C, gearbeitet werden.

Der angewandte Druck ist im allgemeinen für das erfindungsgemäße Verfahren nicht kritisch, es wird daher zweckmäßigerweise bei Atmosphärendruck oder unter dem Eigendruck des Reaktionssystems gearbeitet.

Das Molverhältnis der Summe der Aldehyde R^{a}CHO II und R^{b}CHO III zum Katalysator kann bei sämtlichen Ausgestaltungen des erfindungsgemäßen Verfahrens in einem Bereich von 10:1 bis 10000:1 liegen. Bei Aldehyd/Katalysator-Molverhältnissen von mehr als 200 kann vorteilhaft eine Base oder ein Puffer zum Abfangen von Säuren zugesetzt werden, die sich als Produkt von Nebenreaktionen bilden können. Bei der Herstellung cyclischer Acyloine aus den entsprechenden Dialdehyden wird im allgemeinen ebenfalls ein Molverhältnis Dialdehyd/Katalysator von 10:1 bis 10000:1 angewandt.

Bei der Herstellung gekreuzter Acyloine aus verschiedenen Aldehyden R^{a}CHO und R^{b}CHO wird im allgemeinen ein Molverhältnis von R^{a}HO/R^{b}HO von 1:1 bis 10:1, vorzugsweise ein Molverhältnis von 1:1 angewandt.

Wie bereits erwähnt, wird für die Durchführung des erfindungsgemäßen Verfahrens nach Verfahrensvariante β) oder γ) grundsätzlich keine Hilfsbase benötigt, da die Katalysatoren entweder in situ (Variante β)) oder ex situ (Variante γ)) durch Eliminierung von RXH, vorzugsweise durch die Eliminierung von Methanol, aus den Vorläuferverbindungen V erzeugt werden. Da jedoch während der Umsetzung als Folge von Nebenreaktionen in geringem Ausmaß Säuren entstehen können, beispielsweise durch die Disproportionierung des Aldehyds, und diese Säuren den Katalysator desaktivieren können, kann der Zusatz geringer Mengen an Base, beispielsweise von solchen Basen wie sie zuvor für die Verwendung als Hilfsbasen geeignet genannt wurden, vorteilhaft sein, um diese Säuren abzupuffern oder zu neutralisieren. Dies kann insbesondere dann günstig sein, wenn man mit sehr kleinen Katalysatormengen arbeitet. Es können somit auch in diesen Fällen, die Mengen an Base verwendet werden, wie sie zuvor für Variante α) genannt wurden. Vorteilhaft werden aber geringere Basenmengen eingesetzt. Sind die eingesetzte Aldehyde oder die Aldehyd-liefernden Verbindungen mit Säuren, verunreinigt, so werden diese aus den obengenannten Gründen zweckmäßigerweise vor dem Einsatz im erfindungsgemäßen Verfahren neutralisiert oder die Wirkung dieser Säuren im erfindungsgemäßen Verfahren in situ durch Zusatz einer äquivalenten Basenmenge kompensiert.

Die im erfindungsgemäßen Verfahren verwendeten Triazoliumsalze IV können nach bekannten Verfahren, beispielsweise durch die Alkylierung mesoionischer Verbindungen, beispielsweise die N-Alkylierung von Nitron mittels Alkylhalogeniden oder Dialkylsulfaten (vgl. Busch et al.: Ber 38, 4049 (1905)), durch die oxidative Entschwefelung der entsprechend substituierten Triazolin-5-thione (vgl. Z. Naturforsch. B, 25, 1421 (1970); J. Prakt. Chem., 330, 325 (1988)), welche wiederum durch die Lewis-Säure katalysierte Cyclisierung der entsprechenden Thiosemicarbazone, die aus den entsprechenden Isothiocyanaten nach deren Umsetzung mit einem Alkyl- oder Arylhydrazin zum betreffenden Thioharnstoff und dessen Alkylierung mit einem Aldehyd hergestellt werden können, erhältlich sind (Ber. 42, 4596 (1909); Ber. 34, 320 (1901)). Triazoliumsalze IV, in denen R⁴ eine über das Heteroatom an den Triazoliumring gebundene Ether- oder Thioethergruppe ist, werden ebenfalls ausgehend von den entsprechenden Isocyanaten bzw. Isothiocyanaten durch deren Umsetzung mit einem Hydrazin zum entsprechenden Harnstoff- bzw. Thioharnstoffderivat, dessen Formylierung und Cyclisierung mit Ameisensäure und die anschließende Alkylierung des dabei erhaltenen 1,2,4-Triazolin-5-ons bzw. 1,2,4-Triazolin-5-thions mit einem Alkylierungsmittel synthetisiert (vgl. Ber. 42, 4596 (1909); J. Prakt. Chem. 67, 246 (1903); J. Prakt. Chem. 67, 263 (1903).

Polycyclische Triazoliumsalze IV können aus sekundären Methylaminen nach deren N-Nitrosierung (Org. Synth., Coll. Vol. 2, 460 (1943)) und O-Alkylierung zum entsprechenden Alkoxi-diazeniumsalz in einer 1,3-dipolaren Cycloaddition mit den betreffenden N-Heterocyclen (s. Chem. Ber. 102, 3159 (1969)) hergestellt werden.

Triazoliumsalze IV, die in 3-Position ein Wasserstoffatom tragen, können z.B. nach dem Verfahren von US-A-3 488 761 durch die Umsetzung von 1,2,4-Triazol mit Alkylierungs- oder Arylierungsmitteln, wie Alkylhalogeniden oder Dialkylsulfaten oder Arylhalogeniden, insbesondere Arylfluoriden, erhalten werden. Das 1,2,4-Triazol ist z.B. nach dem Verfahren von Ainsworth et al. J. Am. Chem. Soc. 77, 621 (1955) erhältlich. Alternativ können 3(H)-Triazoliumsalze IV nach dem Verfahren von Boyd et al. J. Chem. Soc (C) 409 (1971) durch die Umsetzung der entsprechenden Oxadiazoliumsalze mit einem primären Amin erhalten werden.

Zur Erzeugung der Additionsverbindungen V setzt man im allgemeinen das betreffende Triazoliumsalz IV in einem Lösungsmittel, beispielsweise einem Ether wie Tetrahydrofuran, Dioxan oder Dimethoxyethan oder einem Alkanol mit dem betreffenden Alkanolat-bzw. Thiolat RXMe um, in dem Me das Grammäquivalent eines Metallkations, vorzugsweise eines Alkalimetallkations, ist und X und R die zuvor genannte Bedeutung haben. Vorzugsweise werden die Alkanolate in dem Alkohol gelöst eingesetzt, aus dem sie erzeugt worden sind. Bei der Umsetzung der Alkanolate oder Thiolate RXMe mit dem Triazoliumsalz IV arbeitet man im allgemeinen bei Temperaturen von 0 bis 100°C, vorzugsweise von 20 bis 50°C und wendet ein Molverhaltnis Alkoholat bzw. Thiolat/Triazoliumsalz IV von im allgemeinen 0,8 bis 1,5, vorzugsweise von 1 bis 1,2 an. Die Additionsprodukte V können aus der so bereiteten Reaktionsmischung nach Entfernung des verwendeten Lösungsmittels und Abtrennung der entstandenen, in organischen Lösungsmitteln schwer löslichen Metallsalze, z.B. durch Filtration, isoliert werden und als solches erfindungsgemäß verwendet werden.

Zum Zwecke der Herstellung des Ylid/Carbens VI/VII werden die Additionsverbindungen V in Substanz oder in einem inerten, hochsiedenden Lösungsmittel z.B. einem Paraffin oder einem Sulfon, wie Sulfolan (Tetrahydrothiophen-1,1-dioxid), auf Temperaturen von 50 bis 160°C, vorzugsweise von 60 bis 140°C und besonders bevorzugt von 70 bis 120°C solange erhitzt, bis sämtliches Alkanol oder Thiol RXH aus V abgespalten ist. Dabei kann unter dem Eigendruck des Reaktionssystems gearbeitet werden, vorzugsweise arbeitet man jedoch bei vermindertem Druck. Das bei dieser Thermolyse erhaltene Thermolyseprodukt kann direkt als Katalysator im erfindungsgemäßen Verfahren eingesetzt werden.

In Anbetracht dessen, daß bei der Selbstkondensation von Formaldehyd mit Triazoliumkatalysatoren IV ein völlig anderes Produktspektrum gebildet wird als bei Verwendung von Thiazolium-Katalysatoren, ein Sachverhalt der auf sterische Hinderungen und Reaktivitätsunterschiede zwischen Triazolium- und Thiazolium-katalysatoren zurückgeführt wurde, ist es sehr überraschend, daß mit den erfindungsgemäßen Katalysatoren auch andere Aldehyde als Formaldehyd zu Acyloinen umgesetzt werden können.

### Beispiele

### Herstellung der Katalysatoren

Die Triazoliumsalze IV A, IV B, IV C und IV D wurden nach Eicher et al (Chem. Ber. 102, 3159 (1969)) hergestellt.

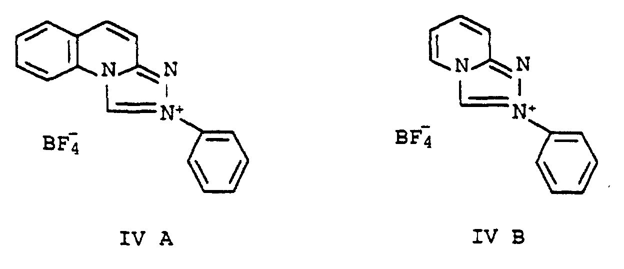

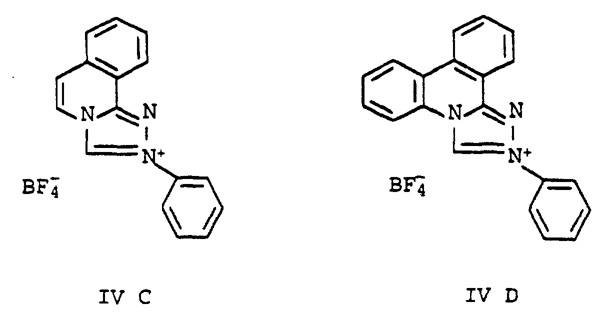

Das Triazoliumsalz IV E wurde, ausgehend von N,N'-Diphenyl-N-aminothioharnstoff (hergestellt nach: Ber. 42, 4596 (1909)) hergestellt:

Zu 20 g (82 mmol) N,N'-Diphenyl-N-aminothioharnstoff wurden langsam und unter Kühlung von außen 3,6 g (82 mmol) frisch destillierter Acetaldehyd gegeben. Dabei wurde die Reaktionsmischung erst zähflüssig, dann fest.

Das so erhaltene Produkt wurde nicht weiter gereinigt, sondern in 400 ml Ethanol aufgenommen und bei 60°C mit einer Lösung von 19,7 g (96 mmol) Eisen(III)chlorid in 50 ml Ethanol versetzt. Nach beendeter Zugabe wurde noch 1 h bei 60°C gerührt und anschließend das Ethanol bei vermindertem Druck abdestilliert. Der Rückstand wurde in Diethylether gelöst, und die etherische Phase mehrfach mit Wasser gewaschen und über Natriumsulfat getrocknet. Das Lösungsmittel wurde abdestilliert, und der Rückstand wurde aus Petroleumbenzin umkristallisiert.

1,4-Diphenyl-3-methyl-1,2,4(5H)-triazolin-5-thion: Massenspektrum: Molpeak 267 m/e
Schmelzpunkt: 128 bis 130°C
Korrekte ¹H- und ¹³C-NMR-Spektren.

4,0 g (15 mmol) 1,4-Diphenyl-3-methyl-1,2,4(5H)-triazolin-5-thion wurden mit 60 ml konzentrierter Salpetersäure, 60 ml Wasser und 14 ml konzentrierter Perchlorsäure bei Raumtemperatur gerührt. Nach wenigen Minuten Reaktionsdauer begann ein Feststoff auszufallen. Nach beendeter Reaktion wurde der Feststoff abfiltriert, nacheinander mit Wasser, Ethanol und Diethylether gewaschen und bei vermindertem Druck getrocknet.

1,4-Diphenyl-3-methyl-1,2,4-triazolium-perchlorat IV E: Schmelzpunkt: 218°C (Zersetzung)
Korrekte ¹H- und ¹³C-NMR-Spektren.

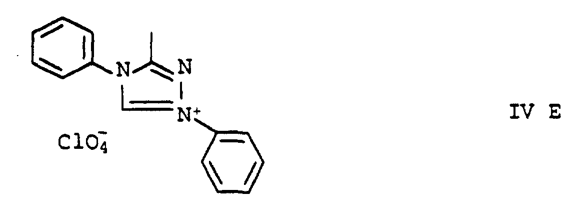

Das Triazoliumsalz IV F wurde nach den in der Beschreibung angegebenen Literaturverfahren über (Z. Naturforsch. B, 25, 1421 (1970); J. Prakt. Chem., 330, 325 (1988)) den Weg des 2,3,4-Triphenyl-substituierten 1,2,4-Triazolin-5-thion-derivats hergestellt.

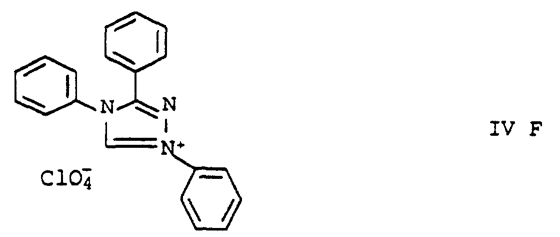

1,3,4-Triphenyl-5-hydroxymethylen-1,2,4-triazolium-perchlorat IV G:
5,0 g (12,5 mmol) IV F, 4,0 g (133 mmol) Paraformaldehyd und 130 ml Tetrahydrofuran wurden 1 h im Glasautoklaven auf 80°C erhitzt. Nach dem Abkühlen wurde das Lösungsmittel bei vermindertem Druck abdestilliert und der halbfeste Rückstand mit 10 gew.-%iger Perchlorsäure gerührt. Der fest gewordene Rückstand wurde mit Wasser neutral gewaschen und aus Ethanol umkristallisiert. Schmelzpunkt: 182 bis 183°C,
Korrekte ¹H- und ¹³C-NMR-Sprektren

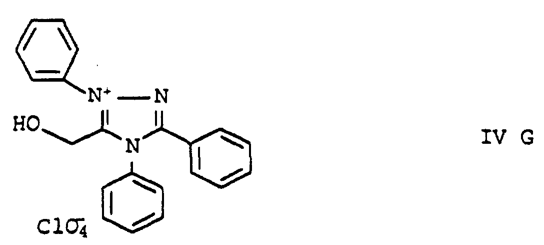

2,4-Diphenyl-5-(N-methyl-N-phenylamino)-1,2,4-triazolium-iodid IV H:
Nitron wurde nach Ber. 38, 4049 (1905) hergestellt und nach dem dort angegebenen Verfahren mit Methyliodid zu IV H umgesetzt.

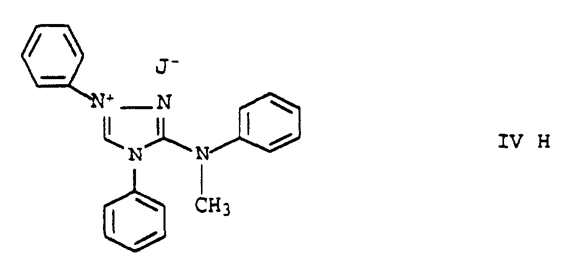

2,0 g IV H (4,4 mmol) wurden in 40,0 g Tetrahydrofuran bei 55°C in einer Argonatmosphäre mit 5,3 g (176 mmol) Paraformaldehyd gelöst und 5 h unter Rückfluß gekocht. Der sich hierbei abscheidende Feststoff wurde in der Wärme abfiltriert. Die organische Lösung wurde mit 100 ml Dichlormethan verdünnt, mehrmals mit Wasser gewaschen und über Magnesiumsulfat getrocknet. Nach Abtrennung des Lösungsmittels bei vermindertem Druck wurde 1,4-Diphenyl-5-hydroxymethylen-3-(N-methyl-N-phenylamino)-1,2,4-triazoliumiodid IV I erhalten.

Schmelzpunkt: 200°C;
Korrekte ¹H- und ¹³C-NMR-Spektren

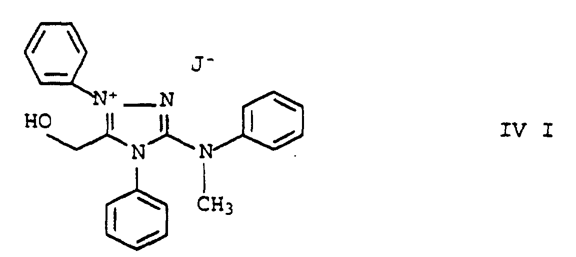

1,4-Diphenyl-3-(N-dodecyl-N-phenylamino)-1,2,4-triazolium-iodid IV J:
2,0 g (6,4 mmol) Nitron und 9,5 g (32 mmol) Dodecyliodid wurden in 10 ml Toluol 24 h unter Argon auf 100°C erhitzt. Aus der abgekühlten Lösung wurde das ausgefallene, kristalline Produkt abfiltriert und aus Ethanol/Wasser umkristallisiert.
Schmelzpunkt: 129 bis 130°C;
Korrekte ¹H- und ¹³C-NMR-Spektren 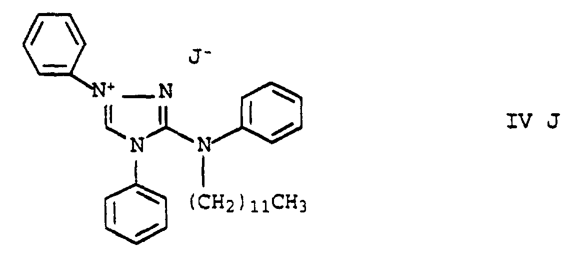 1,4-Diphenyl-3-methoxy-1,2,4-triazolium-tetrafluoroborat IV K

2,0 g (8,4 mmol) 1,4-Diphenyl-3-hydroxy-1,2,4-triazoliumhydroxidinneres Salz (hergestellt nach: J. Prakt. Chem. 67, 263 (1903)) wurden in 50 ml Dichlormethan suspendiert. Zu dieser Suspension wurden bei 0°C 1,3 g (9,2 mmol) festes Trimethyloxoniumtetrafluoroborat langsam gegeben. Die Reaktionsmischung wurde noch 4 h bei 0°C gerührt. Anschließend wurde der Feststoff abfiltriert, mit Dichlormethan gewaschen und aus Ethanol umkristallisiert. Man erhielt IV K in Form eines Gemisches, das zu 58 Gew.-% aus IV K, zu 23 Gew.-% aus der Ausgangsverbindung und zu 19 % aus dem Isomeren 1,4-Diphenyl-2-methyl-1,2,4-(3H)-triazolinium-3-on bestand.

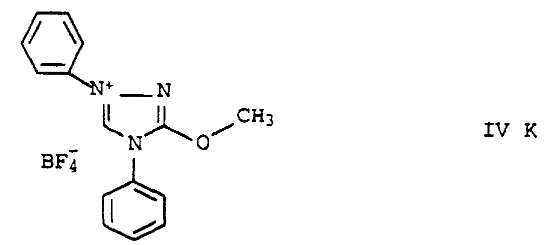

Verbindung IV L wurde nach dem Verfahren von J. Prakt. Chem. 67, 246 (1903) hergestellt. 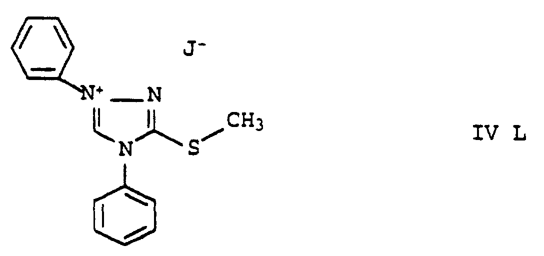 1,3,4-Triphenyl-5-H-5-methoxy-1,2,4-triazolin Va

Zu einer Lösung von 7,0 g (25 mmol) IV F in 150 ml Methanol wurden bei Raumtemperatur 1,4 g (26 mmol) Natriummethanolat, gelöst in 30 ml Methanol, gegeben. Das Methanol wurde anschließend unter vermindertem Druck abgezogen und der Rückstand in Diethylether aufgenommen. Das ungelöst bleibende Natriumperchlorat wurde abfiltriert. Nach Entfernung des Lösungsmittels wurde der zurückbleibende Feststoff aus Methanol umkristallisiert.

Schmelzpunkt: 136 bis 137°C;
Korrekte ¹H- und ¹³C-NMR-Spektren
Ylid/Carben-VIa/VIIa 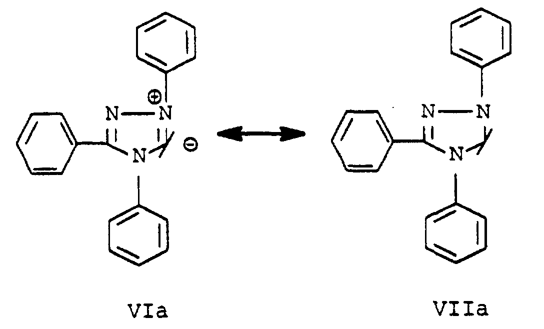 1,0 g (3 mmol) Va wurden solange bei 80°C und bei vermindertem Druck erhitzt, bis der für die Methanolabspaltung berechnete Gewichtsverlust eingetreten war. Dies war nach 16 h der Fall.
1-p-Nitrophenyl-4-methyl-1,2,4-triazolium-jodid IV M

5,0 g (2,6 mmol) 2-p-Nitrophenyl-1,2,4-triazol, 4,1 g (29 mmol) Methyljodid und 20 ml DMF wurden 24 h bei 100°C im Glasautoklaven gerührt. Nach Entfernung des Lösungsmittels wurde der Rückstand in 100 ml Dichlormethan aufgenommen, 30 min gerührt und der ausgefallene Feststoff abfiltriert.
Schmelzpunkt: 213 bis 216°C
Korrekte ¹H- und 13-C-NMR-Spektren

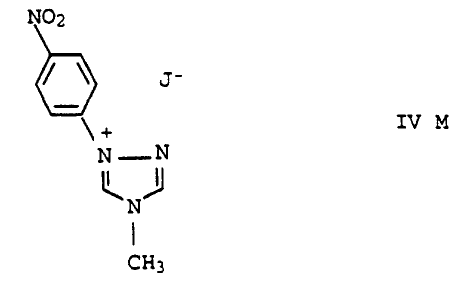

### Beispiel 1

Eine Mischung aus 152 g n-Heptanal und 1,52 g (1 Gew.-%) 5-Methoxy-1,3,4-triphenyl-4,5-dihydro-1,2,4-triazol (Va) wurde unter Eigendruck 2 h auf 130°C erhitzt. Der Umsatz betrug laut gaschromatographischer Analyse 61 % und die Selektivität zu 8-Hydroxy-7-tetradecanon 99 %.

### Beispiele 2 bis 8

Die Beispiele 2 bis 8 wurden analog Beispiel 1 unter Variation von Reaktionstemperatur und Reaktionszeit durchgeführt. Die Ergebnisse dieser Versuche sind in der folgenden Tabelle aufgelistet.

| Beispiel | Temperatur [°C] | Zeit [min] | Umsatz [%] | Selektivität [%] |
|---|---|---|---|---|
| 2 | 110 | 120 | 40 | 95 |
| 3 | 120 | 120 | 53 | 96 |
| 4 | 130 | 60 | 48 | 98 |
| 5 | 130 | 120 | 61 | 99 |
| 6 | 140 | 120 | 59 | 94 |
| 8 | 150 | 120 | 58 | 92 |

### Beispiel 9

1,44 kg n-Butyraldehyd und 13 g Katalysator Va wurden in einem Autoklaven unter Eigendruck 2 h auf 130°C erhitzt. Nach GC-Analyse des Reaktionsaustrags betrug der Umsatz 50 % und die Selektivität zu Butyroin 94 %. Der Reaktionsaustrag wurde mittels Vakuumdestillation aufgearbeitet. Es wurden dabei 673 g Butyroin (5-Hydroxy-4-octanon) erhalten, entsprechend einer Ausbeute von 47 %.

### Beispiele 10 bis 16

Analog Beispiel 1 wurden die folgenden Edukt-Aldehyde mit 1 Gew.-% des Katalysators Va, bezogen auf eingesetztes Edukt, 2 h auf 130°C erhitzt. Der Umsatz (U) und die Selektivität (S) der Umsetzung wurden gaschromatographisch bestimmt.

| Beispiel | Edukt | Produkt | U/S [%] |
|---|---|---|---|
| 10 | Acetaldehyd | Acetoin | 90/95 |
| 11 | Glyoxal-mono-Neopentylglykolacetal | 2-Oxo-3-hydroxy-succinaldehyd-bis-neopentylglykolacetal | 70/66 |
| 12 | 5-Formylvalerian säuremethylester | 6-Hydroxy-7-dodecanon-dicarbon säure-dimethylester | 60/80 |
| 13 | Benzaldehyd | Benzoin | 47/98 |
| 14 | p-Anisaldehyd | 4,4'-Dimethoxybenzoin | 22/90 |
| 15 | Furfurol | Furoin | 89/93 |
| 16 | Pyridin-2-aldehyd | 2,2'-Pyridoin | 84/85 |

### Beispiel 17

89 g (0,5 mol) Glyoxal-mono-neopentylglykolacetal in Form des Halbacetals mit n-Butanol wurden mit 1,64 g (1 mol-%) des Katalysators Va versetzt und durch diese Lösung bei 25°C während einer Zeitdauer von 20 min ein Stickstoffstrom geleitet. Anschließend wurde bei 80°C unter Rühren 2 h lang umgesetzt. Bei einem Umsatz von 70 % betrug die Selektivität für 2-Oxo-3-hydroxy-succinaldehyd-bis-neopentylglykolacetal 92 %.

### Beispiel 18

10 g (0,1 mol) Acetoxyacetaldehyd wurden in 50 g Tetrahydrofuran gelöst und durch diese Lösung für eine Zeitdauer von 20 min ein Stickstoffstrom geleitet. Nach Zugabe von 0,33 g (1 mol-%) des Katalysators Va wurde die Lösung 6 h unter Rückfluß gekocht. Bei einem Umsatz von 55 % betrug die Selektivität für 1,4-Diacetoxy-3-hydroxy-2-butanon 12 % und für 1,3,4-Triacetoxy-2-butanon 36 %.

### Beispiel 19

30 g (0,25 mol) Glyoxylsäuremethylester in Form seines Halbacetals mit Methanol wurden mit 150 g Tetrahydrofuran und 0,82 g (1 mol-%) des Katalysators Va versetzt und 2 h unter einer Stickstoffatmosphäre unter Rückfluß erhitzt. Bei einem Umsatz von 90 % betrug die Selektivität für Dihydroxymaleinsäuredimethylester, der Enol-Form des Acyloins, 99 %. Nach dem Abkühlen kristallisierte das Produkt in 30 % Ausbeute aus.
Schmelzpunkt: 165°C

### Beispiel 20

Eine Mischung aus 10 g Furfurol und 0,1 g Katalysator Va wurde 1 h bei 20°C gerührt. Dabei wurde die Reaktionsmischung kristallin. Bei einem Furfurol-Umsatz von 93 % betrug die Selektivität für Furoin 95 %.

### Beispiel 21

300 ml (336,8 g) einer ca. 50 %igen wäßrigen Glutardialdehyd-Lösung und 300 ml Cyclohexan wurden mittels eines Wasserabscheiders durch azeotrope Destillation entwässert. Nach Abziehen des restlichen Cyclohexans am Rotationsverdampfer wurde der Rückstand aus wasserfreiem Glutardialdehyd (159,9 g =̂ 1,59 mol) in 700 ml Acetonitril gelöst. Zu dieser Lösung wurden 5,23 g (15,9 mmol) Katalysator Va gegeben und die Mischung 5 h unter Rückfluß gekocht. Anschließend wurde das Lösungsmittel am Rotationsverdampfer entfernt und der Rückstand bei vermindertem Druck destilliert. Es wurden 103,2 g (64,9 % Ausbeute) 2-Hydroxycyclopentanon XIII erhalten.

### Beispiel 22

89 g (0,5 mol) Glyoxal-mono-neopentylglykolacetal wurden in Form des Halbacetals mit n-Butanol, mit 1,64 g des Katalysators Va und 15 g (0,5 mol) Formaldehyd versetzt. Ein Stickstoffstrom wurde während 20 min durch die Lösung geleitet. Anschließend wurde 2 h bei 80°C gerührt. Bei einem Umsatz von 75 % betrug die Selektivität für die Mischung der Neopentylglykolacetale von 2-Oxo-3-hydroxy-propionaldehyd und 2-Hydroxy-3-oxo-propionaldehyd 30 %.

### Beispiel 23

Eine Mischung aus 140 g (1,33 mol) Benzaldehyd und 20 g (0,66 mol) Paraformaldehyd wurde unter einer Stickstoffatmosphäre mit 0,54 g des Katalysators Va versetzt und 90 min auf 80°C erhitzt. Bei einem Benzaldehyd-Umsatz von 45 % betrug die Selektivität für 2-Hydroxy-2-phenylacetaldehyd 80 %.

### Beispiel 24

2,9 g (0,03 mol) Furfurol und 10,6 g (0,15 mol) n-Butyraldehyd wurden unter einer Stickstoffatmosphäre mit 0,13 g des Katalysators Va versetzt und 1 h im Autoklaven auf 130°C erhitzt. Bei einem Furfurol-Umsatz von 100 % betrug die Selektivität für die Bildung des Gemisches aus 1-(1-FuryD-1-hydroxy-2-pentanon und 1-(1-Furyl)-2-hydroxy-1-pentanon 60 %.

## Patentansprüche

1. Verfahren zur Herstellung von Acyloinen der allgemeinen Formel I 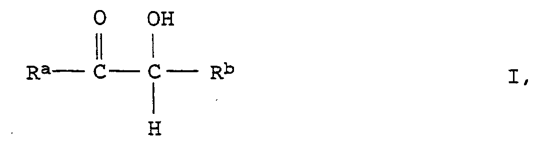 in der R^{a} und R^{b} gleich oder verschieden sind und für Wasserstoff oder eine gegebenenfalls substituierte C₁- bis C₂₀-Alkyl-, eine gegebenenfalls substituierte C₆- bis C₁₀-Aryl-, eine gegebenenfalls substituierte C7- bis C₁₂-Aralkyl-, eine gegebenenfalls substituierte Heteroaryloder eine gegebenenfalls substituierte Heterocycloalkylgruppe stehen, ausgenommen Autokondensationsprodukte des Formaldehyds, **dadurch gekennzeichnet, daß** man einen Aldehyd der Formel II
R^{a}CHO II
mit einem Aldehyd der Formel III
R^{b}CHO III,
in denen R^{a} und R^{b} die obengenannte Bedeutung haben und wenigstens einer der Reste R^{a} und R^{b} einen anderen Rest als Wasserstoff bedeutet, in Gegenwart von Katalysatoren umsetzt, die aus Triazoliumsalzen der Formel IV 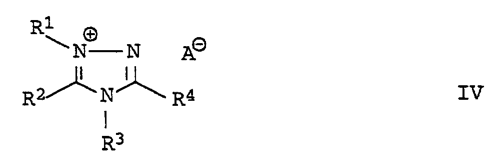 in der
R¹ und R³ gleich oder verschieden sind und für aliphatische Gruppen mit 1 bis 30 Kohlenstoffatomen, C₃- bis C₂₀-Cycloalkyl- oder -alkenylgruppen, C₃- bis C₂₀-Heterocycloalkyloder -alkenylgruppen, über ein Kohlenstoffatom an den Triazoliumring gebundene C₂- bis C₃₀-Alkoxygruppen mit einem oder mehreren Sauerstoffatomen in der Etherkette, C₁- bis C₃₀-Fluor, Chlor- und/oder Brom enthaltende Halogenalkylgruppen mit einem oder mehreren Halogenatomen, über ein Kohlenstoffatom an den Triazoliumring gebundene C₂- bis C₃₀-sekundäre Aminogruppen oder C₃- bis C₃₀-tertiäre Aminogruppen, für gegebenenfalls substituierte Arylgruppen, für gegebenenfalls substituierte Aralkylgruppen und/oder für gegebenenfalls substituierte Heteroarylgruppen stehen,
R² Wasserstoff oder die Gruppe R^{b}CH(OH) ist und in der
R⁴ den Resten R¹ und R³ gleich oder verschieden ist oder Wasserstoff, ein Halogenatom, eine Nitro- oder Cyanogruppe, eine aliphatische Gruppe mit 1 bis 30 Kohlenstoffatomen, C₃- bis C₂₀-Cycloalkyl- oder -alkenylgruppen, C₃- bis C₂₀-Heterocycloalkyl- oder -alkenylgruppen, über ein Kohlenstoffatom an den Triazoliumring gebundene C₂- bis C₃₀-Alkoxygruppen mit einem oder mehreren Sauerstoffatomen in der Etherkette, C₁- bis C₃₀-Fluor, Chlor- und/oder Brom enthaltende Halogenalkylgruppen mit einem oder mehreren Halogenatomen, über ein Kohlenstoffatom an den Triazoliumring gebundene C₂- bis C₃₀-sekundäre Aminogruppen oder C₃- bis C₃₀-tertiäre Aminogruppen, eine gegebenenfalls substituierte Arylgruppe, eine gegebenenfalls substituierte Aralkylgruppe, eine gegebenenfalls substituierte Heteroarylgruppe, eine über das Sauerstoffatom an den Triazoliumring gebundene Alkoxygruppe-OR⁵, eine über das Schwefelatom an den Triazoliumring gebundene Thioethergruppe -SR⁶, eine über das Stickstoffatom an den Triazoliumring gebundene Aminogruppe -NR⁷R⁸, eine Acylgruppe COR⁹ oder eine Estergruppe -COOR¹⁰ bedeutet, wobei die Reste R⁵, R⁶, R⁷, R⁸ und R⁹ für Reste stehen, wie sie für R¹ genannt wurden und R¹⁰ eine C₁- bis C₃₀-Alkyl- oder eine gegebenenfalls substituierte Aryl- oder Aralkylgruppe ist,
oder in der der Rest R³ gemeinsam mit dem Rest R⁴ eine C₃-bis C₅-Alkylen- oder Alkenylen- oder eine C₆- bis C₁₄-Arylen-, eine C₇- bis C₁₄-Aralkylen- oder C₈- bis C₁₄-Aralkenylen-Brücke bildet, und
A das Äquivalent eines ein- oder mehrfach negativ geladenen Anions zur elektrischen Neutralisierung der Ladung des Triazoliumkations ist,
mit Hilfe einer Hilfsbase erzeugt worden sind.

2. Verfahren nach Anspruch 1, **dadurch gekennzeichnet, daß** man die Aldehyde der Formeln II und III mit Katalysatoren umsetzt, die aus Triazoliumsalzen der Formel IV 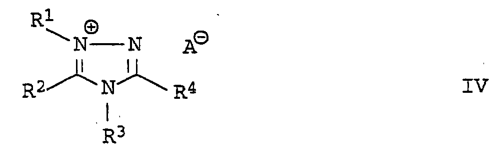 in der
R¹ und R³ gleich oder verschieden sind und für C₁- bis C₃₀-Alkylgruppen, C₂- bis C₃₀-Alkenyl- oder Alkinylgruppen mit 1 oder 2 Mehrfachbindungen, C₃- bis C₂₀-Cycloalkyl- oder -alkenylgruppen, C₃- bis C₂₀-Heterocycloalkyl- oder -alkenylgruppen, über ein Kohlenstoffatom an den Triazoliumring gebundene C₂- bis C₃₀-Alkoxygruppen mit einem oder mehreren Sauerstoffatomen in der Etherkette, C₁- bis C₃₀-Fluor, Chlorund/oder Brom enthaltende Halogenalkylgruppen mit einem oder mehreren Halogenatomen, über ein Kohlenstoffatom an den Triazoliumring gebundene C₂- bis C₃₀-sekundäre Aminogruppen oder C₃- bis C₃₀-tertiäre Aminogruppen, für gegebenenfalls substituierte C₆- bis C₁₄-Arylgruppen, für gegebenenfalls substituierte C₆- bis C₁₄-Aralkylgruppen, für gegebenenfalls substituierte C₇- bis C₂₀-Aralkylgruppen, für gegebenenfalls substituierte C₂- bis C₁₅-Heteroarylgruppen mit 1 bis 3 Stickstoffatomen oder einem Sauerstoff- oder Schwefelatom oder mit 1 bis 2 Stickstoffatomen und einem Sauerstoff- oder Schwefelatom im Ring,
der Rest R⁴ den Resten R¹ oder R³ gleich oder verschieden ist oder ein Wasserstoffatom, eine Nitro- oder Cyanogruppe, ein Fluor-, Chlor- oder Bromatom, eine über das Sauerstoffatom an den Triazoliumring gebundene Alkoxygruppe -OR⁵, eine über das Schwefelatom an den Triazoliumring gebundene Thioethergruppe -SR⁶, eine über das Stickstoffatom an den Triazoliumring gebundene Aminogruppe -NR⁷R⁸, eine Acylgruppe -COR⁹ oder eine Estergruppe -COOR¹⁰ bedeutet, wobei die Reste R⁵, R⁶, R⁷, R⁸ und R⁹ für Reste stehen, wie sie für R¹ genannt wurden und R¹⁰ eine C₁- bis C₃₀-Alkyl- oder eine gegebenenfalls substituierte Aryl- oder Aralkylgruppe ist,
oder in der der Rest R⁴ gemeinsam mit dem Rest R³ eine C₃-bis C₅-Alkylen- oder Alkenylen- oder eine C₆- bis C₁₄-Arylen-, eine C₇- bis C₁₄-Aralkylen- oder C₈- bis C₁₄-Aralkenylen-Brücke bildet, und
in der R² und A die in Anspruch 1 genannte Bedeutung haben,
mit Hilfe einer Hilfsbase erzeugt worden sind.

3. Verfahren nach Anspruch 1, **dadurch gekennzeichnet, daß** man Katalysatoren verwendet, die durch die Eliminierung von Verbindungen RXH aus Verbindungen der Formel V 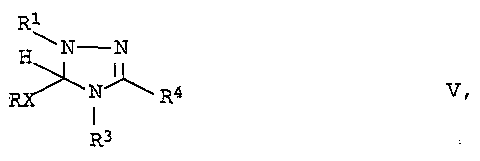 in der X für Sauerstoff oder Schwefel steht und R eine C₁- bis C₄-Alkylgruppe ist und R¹, R³ und R⁴ die in Anspruch 1 genannte Bedeutung haben, erzeugt worden sind.

4. Verfahren nach Anspruch 1, **dadurch gekennzeichnet, daß** man Katalysatoren verwendet, die durch die Eliminierung von Methanol aus Verbindungen der Formel Va 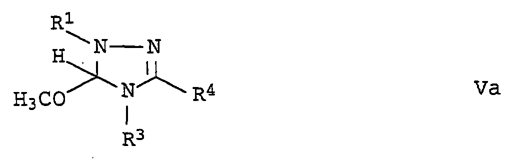 erzeugt worden sind, in der R¹, R³ und R⁴ die in Anspruch 1 genannte Bedeutung haben.

5. Verfahren nach Anspruch 1, **dadurch gekennzeichnet, daß** man der Reaktionsmischung Katalysatoren zusetzt, die vorher in einer separaten Umsetzung durch die thermische Eliminierung von Verbindungen RXH aus Verbindungen der Formel V erzeugt worden sind.

6. Verfahren nach Anspruch 1, **dadurch gekennzeichnet, daß** man der Reaktionsmischung Katalysatoren zusetzt, die vorher in einer separaten Umsetzung durch die thermische Eliminierung von Methanol aus Verbindungen der Formel Va erzeugt worden sind.

7. Verfahren nach Anspruch 1, **dadurch gekennzeichnet, daß** man Aldehyde der Formeln II und III verwendet, die gleiche Reste R^{a} und R^{b} haben.

8. Verfahren nach Anspruch 1, **dadurch gekennzeichnet, daß** man die Aldehyde der Formeln II und III in Form ihrer Halbacetale mit einem aliphatischen oder aromatischen Alkohol verwendet.

9. Verfahren zur Herstellung cycloaliphatischer oder heterocycloaliphatischer Acyloine mit insgesamt 5 bis 12 Ringgliedern, **dadurch gekennzeichnet, daß** man einen gegebenenfalls substituierten aliphatischen C₅- bis C₁₂-Dialdehyd oder einen heteroaliphatischen Dialdehyd mit einer Kettenlänge von 4 bis 11 Kohlenstoffatomen, der zusätzlich in der Kette eine O , S oder N(R^{c}) -Gruppe enthält, in der R^{c} eine C₁- bis C₄-Alkyl- oder Acylgruppe ist, mit einem Katalysator gemäß den Ansprüchen 1 bis 6 umsetzt.

10. Verfahren nach Anspruch 9, **dadurch gekennzeichnet, daß** man die Dialdehyde in Form ihrer Mono- oder Bis-Halbacetale mit einem aliphatischen oder aromatischen Alkohol umsetzt.

11. Verfahren zur Herstellung von gekreuzten Acyloinen der allgemeinen Formel Ia 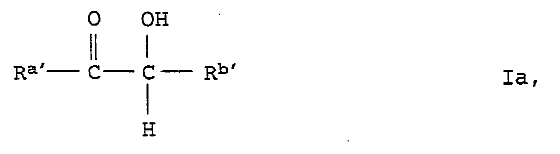 in der die Reste R^{a'} und R^{b'} verschieden sind und für Wasserstoff oder eine gegebenenfalls substituierte C₁- bis C₂₀-Alkyl-, eine gegebenenfalls substituierte C₆- bis C₁₀-Aryl-, eine gegebenenfalls substituierte C₇- bis C₁₁-Aralkyl-, eine gegebenenfalls substituierte Heteroaryloder eine gegebenenfalls substituierte Heterocycloalkylgruppe stehen, ausgenommen Dihydroxyaceton, Glycerinaldehyd sowie C₄- und C₅-Zucker, **dadurch gekennzeichnet, daß** man einen Aldehyd der Formel II
R^{a}CHO II
mit einem Triazoliumsalz der Formel IVa 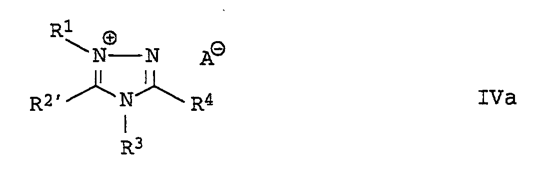 in der
R¹ und R³ gleich oder verschieden sind und für aliphatische Gruppen mit 1 bis 30 Kohlenstoffatomen, C₃- bis C₂₀-Cycloalkyl- oder -alkenylgruppen, C₃- bis C₂₀-Heterocycloalkyloder -alkenylgruppen, über ein Kohlenstoffatom an den Triazoliumring gebundene C₂- bis C₃₀-Alkoxygruppen mit einem oder mehreren Sauerstoffatomen in der Etherkette, C₁- bis C₃₀-Fluor, Chlor- und/oder Brom enthaltende Halogenalkylgruppen mit einem oder mehreren Halogenatomen, über ein Kohlenstoffatom an den Triazoliumring gebundene C₂- bis C₃₀-sekundäre Aminogruppen oder C₃- bis C₃₀-tertiäre Aminogruppen für gegebenenfalls substituierte Arylgruppen, für gegebenenfalls substituierte Aralkylgruppen und/oder für gegebenenfalls substituierte Heteroarylgruppen stehen,
R^{2'} die Gruppe R^{b}CH(OH) darstellt, und in der
R⁴ den Resten R¹ oder R³ gleich oder verschieden ist oder Wasserstoff, ein Halogenatom, eine Nitro- oder Cyanogruppe, eine aliphatische Gruppe mit 1 bis 30 Kohlenstoffatomen, C₃-bis C₂₀-Cycloalkyl- oder -alkenylgruppen, C₃- bis C₂₀-Heterocycloalkyl- oder -alkenylgruppen, über ein Kohlenstoffatom an den Triazoliumring gebundene C₂- bis C₃₀-Alkoxygruppen mit einem oder mehreren Sauerstoffatomen in der Etherkette, C₁-bis C₃₀-Fluor, Chlor- und/oder Brom enthaltende Halogenalkylgruppen mit einem oder mehreren Halogenatomen, über ein Kohlenstoffatom an den Triazoliumring gebundene C₂- bis C₃₀-sekundäre Aminogruppen oder C₃- bis C₃₀-tertiäre Aminogruppen, eine gegebenenfalls substituierte Arylgruppe, eine gegebenenfalls substituierte Aralkylgruppe, eine gegebenenfalls substituierte Heteroarylgruppe, eine über das Sauerstoffatom an den Triazoliumring gebundene Alkoxygruppe -OR⁵, eine über das Schwefelatom an den Triazoliumring gebundene Thioethergruppe -SR⁶, eine über das Stickstoffatom an den Triazoliumring gebundene Aminogruppe -NR⁷R⁸, eine Acylgruppe -COR⁹ oder eine Estergruppe -COOR¹⁰ bedeutet, wobei die Reste R⁵, R⁶, R⁷, R⁸ und R⁹ für Reste stehen, wie sie für R¹ genannt wurden und R¹⁰ eine C₁- bis C₃₀-Alkyl- oder eine gegebenenfalls substituierte Aryl- oder Aralkylgruppe ist,
oder in der der Rest R³ gemeinsam mit dem Rest R⁴ eine C₃-bis C₅-Alkylen- oder Alkenylen- oder eine C₆- bis C₁₄-Arylen-, eine C₇- bis C₁₄-Aralkylen- oder C₈- bis C₁₄-Aralkenylen-Brücke bildet, und
A das Äquivalent eines ein- oder mehrfach negativ geladenen Anions zur elektrischen Neutralisierung der Ladung des Triazoliumkations ist,
in Gegenwart einer Hilfsbase und in einem Molverhältnis Triazoliumsalz IVa/Aldehyd II von 1:1 bis 5:1 umsetzt, mit der Maßgabe, daß mindestens einer der Reste R^{a} oder R^{b} von Wasserstoff verschieden ist.

12. Verfahren nach Anspruch 11, **dadurch gekennzeichnet, daß** man die Aldehyde der Formel II mit einem Triazoliumsalz der Formel IVa 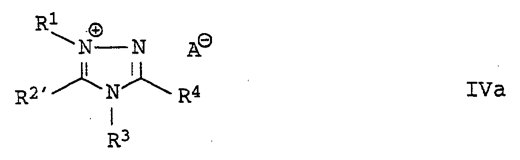 in der
R¹ und R³ gleich oder verschieden sind und für C₁- bis C₃₀-Alkylgruppen, C₂- bis C₃₀-Alkenyl- oder Alkinylgruppen mit 1 oder 2 Mehrfachbindungen, C₃- bis C₂₀-Cycloalkyl- oder alkenylgruppen, C₃- bis C₂₀-Heterocycloalkyl- oder -alkenylgruppen, über ein Kohlenstoffatom an den Triazoliumring gebundene C₂- bis C₃₀-Alkoxygruppen mit einem oder mehreren Sauerstoffatomen in der Etherkette, C₁- bis C₃₀-Fluor, Chlorund/oder Brom-enthaltende Halogenalkylgruppen mit einem oder mehreren Halogenatomen, über ein Kohlenstoffatom an den Triazoliumring gebundene C₂- bis C₃₀-sekundäre Aminogruppen oder C₃- bis C₃₀-tertiäre Aminogruppen, für gegebenenfalls substituierte C₆- bis C₁₄-Arylgruppen, für gegebenenfalls substituierte C₆- bis C₁₄-Aralkylgruppen, für gegebenenfalls substituierte C₇- bis C₂₀-Aralkylgruppen, für gegebenenfalls substituierte C₂- bis C₁₅-Heteroarylgruppen mit 1 bis 3 Stickstoffatomen oder einem Sauerstoff- oder Schwefelatom oder mit 1 bis 2 Stickstoffatomen und einem Sauerstoff- oder Schwefelatom im Ring,
der Rest R⁴ den Resten R¹ oder R³ gleich oder verschieden ist oder ein Wasserstoffatom, eine Nitro- oder Cyanogruppe, ein Fluor-, Chlor- oder Bromatom, eine über das Sauerstoffatom an den Triazoliumring gebundene Alkoxygruppe -OR⁵, eine über das Schwefelatom an den Triazoliumring gebundene Thioethergruppe -SR⁶, eine über das Stickstoffatom an den Triazoliumring gebundene Aminogruppe -NR⁷R⁸, eine Acylgruppe -COR⁹ oder eine Estergruppe -COOR¹⁰ bedeutet, wobei die Reste R⁵, R⁶, R⁷, R⁸ und R⁹ für Reste stehen, wie sie für R¹ genannt wurden und R¹⁰ eine C₁- bis C₃₀-Alkyl- oder eine gegebenenfalls substituierte Aryl- oder Aralkylgruppe ist,
oder in der der Rest R⁴ gemeinsam mit dem Rest R³ eine C₃-bis C₅-Alkylen- oder Alkenylen- oder eine C₆- bis C₁₄-Arylen-, eine C₇- bis C₁₄-Aralkylen- oder C₈- bis C₁₄-Aralkenylen-Brücke bildet, und
in der R²' und A die in Anspruch 11 genannte Bedeutung haben,
in Gegenwart einer Hilfsbase und in einem Molverhältnis Triazoliumsalz IVa/Aldehyd II von 1:1 bis 5:1 umsetzt, mit der Maßgabe, daß mindestens einer der Reste R^{a} oder R^{b} von Wasserstoff verschieden ist.

## Claims

1. A process for preparing acyloins of the formula I 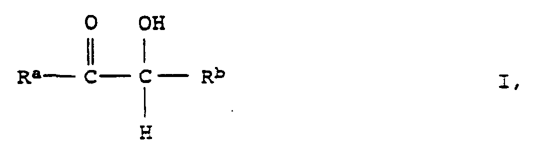 where R^{a} and R^{b} are identical or different and are hydrogen or an unsubstituted or substituted C₁-C₂₀-alkyl, an unsubstituted or substituted C₆-C₁₀-aryl, an unsubstituted or substituted C₇-C₁₂-aralkyl, an unsubstituted or substituted hetaryl or an unsubstituted or substituted heterocycloalkyl group, except formaldehyde autocondensates, which comprises reacting an aldehyde of the formula II
R^{a}CHO II
with an aldehyde of the formula III
R^{b}CHO III
where R^{a} and R^{b} have the abovementioned meaning, and at least one of the radicals R^{a} and R^{b} is a radical different from hydrogen, in the presence of catalysts which have been generated with the aid of a base from triazolium salts of the formula IV 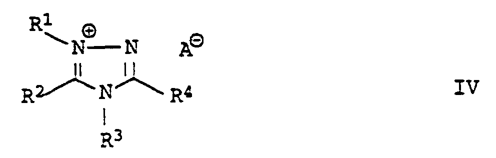 where
R¹ and R³ are identical or different and are aliphatic groups having 1 to 30 carbon atoms, C₃-C₂₀-cycloalkyl or -alkenyl groups, C₃-C₂₀-heterocycloalkyl or -alkenyl groups, or C₂-C₃₀-alkoxy groups which are bonded to the triazolium ring via a carbon atom and which have one or more oxygen atoms in the ether chain, C₁-C₃₀-fluorine-, chlorine- and/or bromine-containing haloalkyl groups having one or more halogen atoms, or C₂-C₃₀-secondary amino groups or C₃-C₃₀-tertiary amino groups, each of which are bonded to the triazolium ring via a carbon atom, or unsubstituted or substituted aryl groups, unsubstituted or substituted aralkyl groups and/or unsubstituted or substituted hetaryl groups,
R² is hydrogen or the group R^{b}CH(OH), and where
R⁴ is identical to or different from the radicals R¹ and R³, or is hydrogen, a halogen atom, a nitro or cyano group, an aliphatic group having 1 to 30 carbon atoms, C₃-C₂₀-cycloalkyl or -alkenyl groups, C₃-C₂₀-heterocycloalkyl or -alkenyl groups, or C₂-C₃₀-alkoxy groups which are bonded to the triazolium ring via a carbon atom and which have one or more oxygen atoms in the ether chain, C₁-C₃₀-fluorine-, chlorineand/or bromine-containing haloalkyl groups having one or more halogen atoms, or C₂-C₃₀-secondary amino groups or C₃-C₃₀-tertiary amino groups, each of which are bonded to the triazolium ring via a carbon atom, or an unsubstituted or substituted aryl group, an unsubstituted or substituted aralkyl group, an unsubstituted or substituted hetaryl group, an alkoxy group -OR⁵ which is bonded to the triazolium ring via the oxygen atom, a thioether group -SR⁶ which is bonded to the triazolium ring via the sulfur atom, an amino group -NR⁷R⁸ which is bonded to the triazolium ring via the nitrogen atom, an acyl group -COR⁹ or an ester group -COOR¹⁰, where the radicals R⁵, R⁶, R⁷, R⁸ and R⁹ are radicals as mentioned for R¹, and R¹⁰ is a C₁-C₃₀-alkyl or an unsubstituted or substituted aryl or aralkyl group, or where the radical R³ forms together with the radical R⁴ a C₃-C₅-alkylene or alkenylene or a C₆-C₁₄-arylene, a C₇-C₁₄-aralkylene or C₈-C₁₄-aralkenylene bridge, and
A is the equivalent of an anion which has one or more negative charges for electrical neutralization of the charge of the triazolium cation.

2. A process as claimed in claim 1, wherein the aldehydes of the formulae II and III are reacted with catalysts which have been generated with the aid of a base from triazolium salts of the formula IV 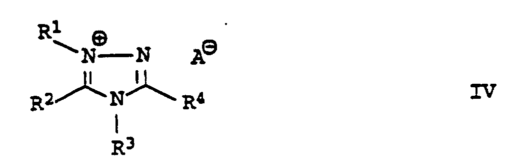 where
R¹ and R³ are identical or different and are C₁-C₃₀-alkyl groups, C₂-C₃₀-alkenyl or alkynyl groups having 1 or 2 multiple bonds, C₃-C₂₀-cycloalkyl or -alkenyl groups, C₃-C₂₀-heterocycloalkyl or -alkenyl groups, or C₂-C₃₀-alkoxy groups which are bonded to the triazolium ring via a carbon atom and which have one or more oxygen atoms in the ether chain, C₁-C₃₀-fluorine-, chlorine- and/or bromine-containing haloalkyl groups having one or more halogen atoms, or C₂-C₃₀-secondary amino groups or C₃-C₃₀-tertiary amino groups, each of which are bonded to the triazolium ring via a carbon atom, or unsubstituted or substituted C₆-C₁₄-aryl groups, unsubstituted or substituted C₆-C₁₄-aralkyl groups, unsubstituted or substituted C₇-C₂₀-aralkyl groups, unsubstituted or substituted C₂-C₁₅-hetaryl groups having 1 to 3 nitrogen atoms or one oxygen or sulfur atom or having 1 to 2 nitrogen atoms and one oxygen or sulfur atom in the ring,
the radical R⁴ is identical to or different from the radicals R¹ or R³ or is a hydrogen atom, a nitro or cyano group, a fluorine, chlorine or bromine atom, an alkoxy group -OR⁵ which is bonded to the triazolium ring via the oxygen atom, a thioether group -SR⁶ which is bonded to the triazolium ring via the sulfur atom, an amino group -NR⁷R⁸ which is bonded to the triazolium ring via the nitrogen atom, an acyl group -COR⁹ or an ester group -COOR¹⁰, where the radicals R⁵, R⁶, R⁷, R⁸ and R⁹ are radicals as have been mentioned for R¹, and
R¹⁰ is a C₁-C₃₀-alkyl or an unsubstituted or substituted aryl or aralkyl group,
or where the radical R⁴ forms together with the radical R³ a C₃-C₅-alkylene or alkenylene or a C₆-C₁₄-arylene, a C₇-C₁₄-aralkylene or C₈-C₁₄-aralkenylene bridge, and
where R² and A have the meanings stated in claim 1.

3. A process as claimed in claim 1, wherein catalysts which have been generated by eliminating compounds RXH from compounds of the formula V 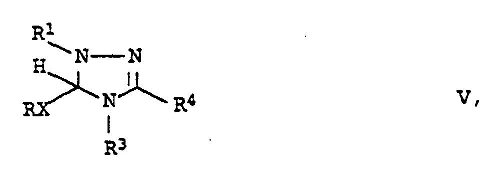 where X is oxygen or sulfur, and R is a C₁-C₄-alkyl group, and R¹, R³ and R⁴ have the meanings stated in claim 1, are used.

4. A process as claimed in claim 1, wherein catalysts which have been generated by eliminating methanol from compounds of the formula Va 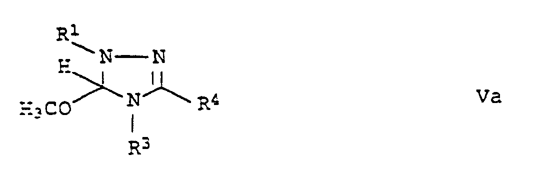 where R¹, R³ and R⁴ have the meanings stated in claim 1, are used.

5. A process as claimed in claim 1, wherein catalysts which have previously been generated in a separate reaction by thermal elimination of compounds RXH from compounds of the formula V are added to the reaction mixture.

6. A process as claimed in claim 1, wherein catalysts which have previously been generated in a separate reaction by thermal elimination of methanol from compounds of the formula Va are added to the reaction mixture.

7. A process as claimed in claim 1, wherein aldehydes of the formulae II and III which have identical radicals R^{a} and R^{b} are used.

8. A process as claimed in claim 1, wherein the aldehydes of the formulae II and III are used in the form of their hemiacetals with an aliphatic or aromatic alcohol.

9. A process for preparing cycloaliphatic or heterocycloaliphatic acyloins having a total of 5 to 12 ring members, which comprises reacting an unsubstituted or substituted aliphatic C₅-C₁₂-dialdehyde or a heteroaliphatic dialdehyde which has a chain length from 4 to 11 carbon atoms and which additionally contains an -O-, -S- or -N(R^{c})- group in the chain, where R^{c} is a C₁-C₄-alkyl or acyl group, with a catalyst as set forth in claims 1 to 6.

10. A process as claimed in claim 9, wherein the dialdehydes are reacted in the form of their mono- or bishemiacetals with an aliphatic or aromatic alcohol.

11. A process for preparing crossed acyloins of the formula Ia 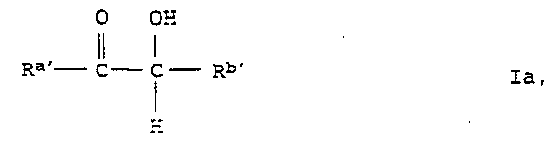 where the radicals R^{a'} and R^{b'} are different and are hydrogen or an unsubstituted or substituted C₁-C₂₀-alkyl, an unsubstituted or substituted C₆-C₁₀-aryl, an unsubstituted or substituted C₇-C₁₁-aralkyl, an unsubstituted or substituted hetaryl or an unsubstituted or substituted heterocycloalkyl group, except dihydroxyacetone, glyceraldehyde and C₄- and C₅-sugars, which comprises reacting an aldehyde of the formula II
R^{a}CHO II
with a triazolium salt of the formula IVa 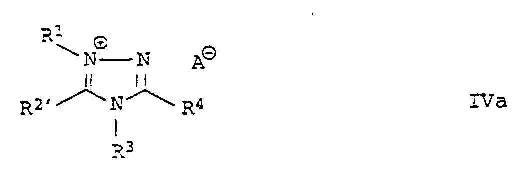 where
R¹ and R³ are identical or different and are aliphatic groups having 1 to 30 carbon atoms, C₃-C₂₀-cycloalkyl or -alkenyl groups, C₃-C₂₀-heterocycloalkyl or -alkenyl groups, or C₂-C₃₀-alkoxy groups which are bonded to the triazolium ring via a carbon atom and which have one or more oxygen atoms in the ether chain, C₁-C₃₀-fluorine-, chlorine- and/or bromine-containing haloalkyl groups having one or more halogen atoms, or C₂-C₃₀-secondary amino groups or C₃-C₃₀-tertiary amino groups, each of which are bonded to the triazolium ring via a carbon atom, or unsubstituted or substituted aryl groups, unsubstituted or substituted aralkyl groups and/or unsubstituted or substituted hetaryl groups,
R²' is the group R^{b}CH(OH), and where
R⁴ is identical to or different from the radicals R¹ or R³, or is hydrogen, a halogen atom, a nitro or cyano group, an aliphatic group having 1 to 30 carbon atoms, C₃-C₂₀-cycloalkyl or -alkenyl groups, C₃-C₂₀-heterocycloalkyl or -alkenyl groups, or C₂-C₃₀-alkoxy groups which are bonded to the triazolium ring via a carbon atom and which have one or more oxygen atoms in the ether chain, C₁-C₃₀-fluorine-, chlorineand/or bromine-containing haloalkyl groups having one or more halogen atoms, or C₂-C₃₀-secondary amino groups or C₃-C₃₀-tertiary amino groups, each of which are bonded to the triazolium ring via a carbon atom, or an unsubstituted or substituted aryl group, an unsubstituted or substituted aralkyl group, an unsubstituted or substituted hetaryl group, an alkoxy group -OR⁵ which is bonded to the triazolium ring via the oxygen atom, a thioether group -SR⁶ which is bonded to the triazolium ring via the sulfur atom, an amino group -NR⁷R⁸ which is bonded to the triazolium ring via the oxygen atom, an acyl group -COR⁹ or an ester group -COOR¹⁰, where the radicals R⁵, R⁶, R⁷, R⁸ and R⁹ are radicals as have been mentioned for R¹, and R¹⁰ is a C₁-C₃₀-alkyl or an unsubstituted or substituted aryl or aralkyl group,
or where the radical R³ forms together with the radical R⁴ a C₃-C₅-alkylene or alkenylene or a C₆-C₁₄-arylene, a C₇-C₁₄-aralkylene or C₈-C₁₄-aralkenylene bridge, and
A is the equivalent of an anion which has one or more negative charges for electrical neutralization of the charge of the triazolium cation,

12. A process as claimed in claim 11, wherein the aldehydes of the formula II are reacted with a triazolium salt of the formula IVa 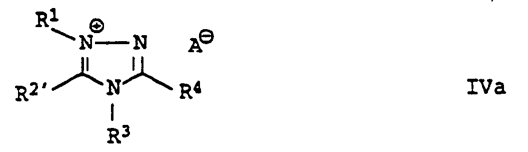 where
R¹ and R³ are identical or different and are C₁-C₃₀-alkyl groups, C₂-C₃₀-alkenyl or alkynyl groups having 1 or 2 multiple bonds, C₃-C₂₀-cycloalkyl or alkenyl groups, C₃-C₂₀-heterocycloalkyl or -alkenyl groups, or C₂-C₃₀-alkoxy groups which are bonded to the triazolium ring via a carbon atom and have one or more oxygen atoms in the ether chain, C₁-C₃₀-fluorine-, chlorine- and/or bromine-containing haloalkyl groups having one or more halogen atoms, or C₂-C₃₀-secondary amino groups or C₃-C₃₀-tertiary amino groups, each of which are bonded to the triazolium ring via a carbon atom, or unsubstituted or substituted C₆-C₁₄-aryl groups, unsubstituted or substituted C₆-C₁₄-aralkyl groups, unsubstituted or substituted C₇-C₂₀-aralkyl groups, unsubstituted or substituted C₂-C₁₅-hetaryl groups having 1 to 3 nitrogen atoms or one oxygen or sulfur atom or having 1 to 2 nitrogen atoms and one oxygen or sulfur atom in the ring,
the radical R⁴ is identical to or different from the radicals R¹ or R³, or is a hydrogen atom, a nitro or cyano group, a fluorine, chlorine or bromine atom, an alkoxy group -OR⁵ which is bonded to the triazolium ring via the oxygen atom, a thioether group -SR⁶ which is bonded to the triazolium ring via the sulfur atom, an amino group -NR⁷R⁸ which is bonded to the triazolium ring via the nitrogen atom, an acyl group -COR⁹, or an ester group -COOR¹⁰, where the radicals R⁵, R⁶, R⁷, R⁸ and R⁹ are radicals as have been mentioned for R¹, and R¹⁰ is a C₁-C₃₀-alkyl or an unsubstituted or substituted aryl or aralkyl group,
or where the radical R⁴ forms together with the radical R³ a C₃-C₅-alkylene or alkenylene or a C₆-C₁₄-arylene, a C₇-C₁₄-aralkylene or C₈-C₁₄-aralkenylene bridge, and
where R^{2'} and A have the meanings stated in claim 11,
in the presence of a base and in a triazolium salt IVa/aldehyde II molar ratio of from 1:1 to 5:1, with the proviso that at least one of the radicals R^{a} or R^{b} is different from hydrogen.

## Revendications

1. Procédé de préparation d'acyloïnes de formule générale I 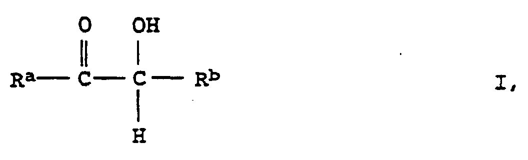 dans laquelle R^{a} et R^{b} sont identiques ou différents et représentent chacun un atome d'hydrogène ou un groupe alkyle en C₁ à C₂₀ éventuellement substitué, un groupe aryle en C₆ à C₁₀ éventuellement substitué, un groupe aralkyle en C₇ à C₁₂ éventuellement substitué, un groupe hétéroaryle éventuellement substitué ou un groupe hétérocycloalkyle éventuellement substitué, à l'exception des produits d'autocondensation du formaldéhyde, **caractérisé en ce qu'**on fait réagir un aldéhyde de formule II
R^{a}CHO II
avec un aldéhyde de formule III
R^{b}CHO III
dans lesquelles R^{a} et R^{b} ont les significations ci-dessus, et au moins l'un des radicaux R^{a} et R^{b} est un radical autre que l'hydrogène, en présence de catalyseurs qui ont été obtenus, à l'aide d'une base auxiliaire, à partir de sels de triazolium de formule IV 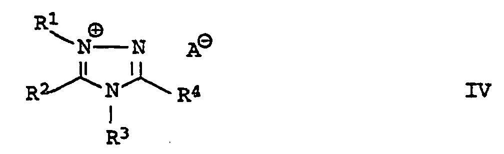 dans laquelle
R¹ et R³ sont identiques ou différents et représentent des groupes aliphatiques ayant de 1 à 30 atomes de carbone, des groupes cycloalkyle ou cycloalcényle en C₃ à C₂₀, des groupes hétérocycloalkyle ou hétérocycloalcényle en C₃ à C₂₀, des groupes alcoxy en C₂ à C₃₀ liés au noyau triazolium par l'intermédiaire d'un atome de carbone et comportant un ou plusieurs atomes d'oxygène dans la chaîne éther, des groupes halogènalkyle en C₁ à C₃₀ contenant du fluor, du chlore et/ou du brome et comportant un ou plusieurs atomes d'halogène, des groupes amino secondaires en C₂ à C₃₀ ou des groupes amino tertiaires en C₃ à C₃₀ liés au noyau triazolium par l'intermédiaire d'un atome de carbone, des groupes aryle éventuellement substitués, des groupes aralkyle éventuellement substitués et/ou des groupes hétéroaryle éventuellement substitués,
R² est un atome d'hydrogène ou le groupe R^{b}CH(OH), et dans laquelle
R⁴ est identique aux radicaux R¹ et R³ ou en est différent, ou encore représente un atome d'hydrogène, un atome d'halogène, un groupe nitro ou cyano, un groupe aliphatique ayant de 1 à 30 atomes de carbone, un groupe cycloalkyle ou cycloalcényle en C₃ à C₂₀, un groupe hétérocycloalkyle ou hétérocycloalcényle en C₃ à C₂₀, un groupe alcoxy en C₂ à C₃₀, lié au noyau triazolium par l'intermédiaire d'un atome de carbone et comportant un ou plusieurs atomes d'oxygène dans la chaîne éther, un groupe halogènalkyle en C₁ à C₃₀ contenant du fluor, du chlore et/ou du brome, et ayant un ou plusieurs atomes d'halogène, un groupe amino secondaire en C₂ à C₃₀ ou un groupe amino tertiaire en C₃ à C₃₀ lié au noyau triazolium par l'intermédiaire d'un atome de carbone, un groupe aryle éventuellement substitué, un groupe aralkyle éventuellement substitué, un groupe hétéroaryle éventuellement substitué, un groupe alcoxy -OR⁵ lié au noyau triazolium par l'intermédiaire de l'atome d'oxygène, un groupe thioéther -SR⁶ lié au noyau triazolium par l'intermédiaire de l'atome de soufre, un groupe amino -NR⁷R⁸ lié au noyau triazolium par l'intermédiaire de l'atome d'azote, un groupe acyle COR⁹ ou un groupe ester -COOR¹⁰, les radicaux R⁵, R⁶, R⁷, R⁸ et R⁹ représentant des radicaux tels ceux qui sont mentionnés pour R¹, et R¹⁰ est un groupe alkyle en C₁ à C₃₀ ou un groupe aryle ou aralkyle éventuellement substitué,
ou bien dans laquelle le radical R³, avec le radical R⁴, forme un pont alkylène ou alcénylène en C₃ à C₅ ou un pont arylène en C₆ à C₁₄, un pont aralkylène en C₇ à C₁₄ ou un pont aralcénylène en C₈ à C₁₄, et
A est l'équivalent d'un anion une ou plusieurs négativement chargé, pour assurer la neutralisation électrique de la charge du cation triazolium.

2. Procédé selon la revendication 1, **caractérisé en ce qu'**on fait réagir les aldéhydes ayant les formules II et III avec des catalyseurs qui ont été préparés à partir de sels de triazolium de formule IV 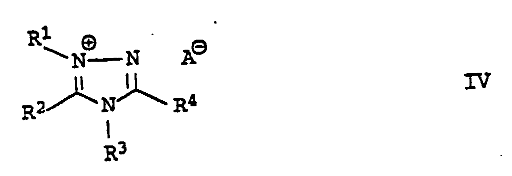 dans laquelle
R¹ et R³ sont identiques ou différents et représentent des groupes alkyle en C₁ à C₃₀, des groupes alcényle ou alcynyle en C₂ à C₃₀ ayant 1 ou 2 liaisons multiples, des groupes cycloalkyle ou cycloalcényle en C₃ à C₂₀, des groupes hétérocycloalkyle ou hétérocycloalcényle en C₃ à C₂₀, des groupes alcoxy en C₂ à C₃₀ liés au noyau triazolium par l'intermédiaire d'un atome de carbone et ayant un ou plusieurs atomes de carbone dans la chaîne éther, des groupes halogènalkyle en C₁ à C₃₀ contenant du fluor, du chlore et/ou du brome, et ayant un ou plusieurs atomes d'halogène, des groupes amino secondaires en C₂ à C₃₀ ou des groupes amino tertiaires en C₃ à C₁₀ liés au noyau triazolium par l'intermédiaire d'un atome de carbone, des groupes aryle en C₆ à C₁₄ éventuellement substitués, des groupes aralkyle en C₆ à C₁₄ éventuellement substitués, des groupes aralkyle en C₇ à C₂₀ éventuellement substitués, des groupes hétéroaryle en C₂ à C₁₅ éventuellement substitués et ayant sur le noyau de 1 à 3 atomes d'azote ou un atome d'oxygène ou un atome de soufre, ou encore ayant 1 à 2 atomes d'azote et un atome d'oxygène ou un atome de soufre,
le radical R⁴ est identique aux radicaux R¹ ou R³ ou en est différent, ou est un atome d'hydrogène, un groupe nitro ou cyano, un atome de fluor, de chlore ou de brome, un groupe alcoxy -OR⁵ lié au noyau triazolium par l'intermédiaire de l'atome d'oxygène, un groupe thioéther -SR⁶ lié au noyau triazolium par l'intermédiaire de l'atome de soufre, un groupe amino -NR⁷R⁸ lié au noyau triazolium par l'intermédiaire de l'atome d'azote, un groupe acyle COR⁹ ou un groupe ester -COOR¹⁰, les radicaux R⁵, R⁶, R⁷, R⁸ et R⁹ représentant des radicaux tels ceux qui sont mentionnés pour R¹, et R¹⁰ est un groupe alkyle en C₁ à C₃₀ ou un groupe aryle ou aralkyle éventuellement substitué,
ou bien dans laquelle le radical R⁴, avec le radical R³, forme un pont alkylène ou alcénylène en C₃ à C₅ ou un pont arylène en C₆ à C₁₄, un pont aralkylène en C₇ à C₁₄ ou un pont aralcénylène en C₈ à C₁₄, et
dans laquelle R² et A ont les significations données dans la revendication 1,
à l'aide d'une base auxiliaire.

3. Procédé selon la revendication 1, **caractérisé en ce qu'**on utilise des catalyseurs qui ont été préparés par élimination de composés RXH à partir de composés de formule V 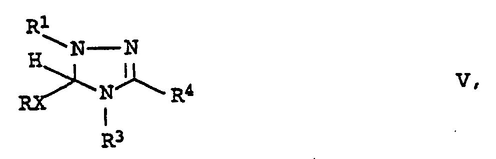 dans laquelle X est un atome d'oxygène ou de soufre, et R est un groupe alkyle en C₁ à C₄, et R¹, R³ et R⁴ ont les significations données dans la revendication 1.

4. Procédé selon la revendication 1, **caractérisé en ce qu'**on utilise des catalyseurs qui ont été préparés par élimination de méthanol à partir de composés de formule Va 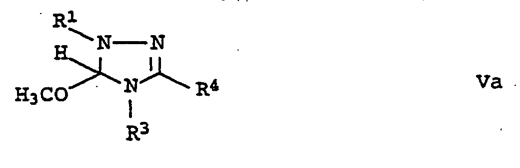 dans laquelle R¹, R³ et R⁴ ont les significations données dans la revendication 1.

5. Procédé selon la revendication 1, **caractérisé en ce qu'**on ajoute au mélange réactionnel des catalyseurs qui ont été préparés au préalable dans le cadre d'une réaction distincte par élimination thermique de composés RXH à partir de composés de formule V.

6. Procédé selon la revendication 1, **caractérisé en ce qu'**on ajoute au mélange réactionnel des catalyseurs qui ont été préparés au préalable dans le cadre d'une réaction distincte, par élimination thermique de méthanol à partir de composés de formule Va.

7. Procédé selon la revendication 1, **caractérisé en ce qu'**on utilise des aldéhydes ayant les formules II et III qui ont les mêmes radicaux R^{a} et R^{b}.

8. Procédé selon la revendication 1, **caractérisé en ce qu'**on utilise les aldéhydes ayant les formules II et III sous forme de leurs semi-acétals avec un alcool aliphatique ou aromatique.

9. Procédé de préparation d'acyloïnes cycloaliphatiques ou hétérocycloaliphatiques ayant en tout de 5 à 12 chaînons, **caractérisé en ce qu'**on fait réagir avec un catalyseur selon les revendications 1 à 6 un dialdéhyde en C₅ à C₁₂ aliphatique éventuellement substitué ou un dialdéhyde hétéroaliphatique ayant une longueur de chaîne de 4 à 11 atomes de carbone, qui contient en outre dans la chaîne un groupe -O-, -S- ou -N(R^{c})-, dans lequel R^{c} est un groupe alkyle ou acyle en C₁ à C₄.

10. Procédé selon la revendication 9, **caractérisé en ce qu'**on fait réagir les dialdéhydes sous forme de leurs mono- ou bis semi-acétals avec un alcool aliphatique ou aromatique.

11. Procédé de préparation d'acyloïnes croisées de formule générale Ia 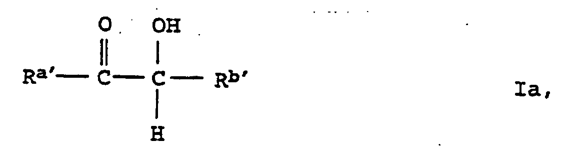 dans laquelle les radicaux R^{a'} et R^{b'} sont différents et représentent chacun un atome d'hydrogène ou un groupe alkyle en C₁ à C₂₀ éventuellement substitué, un groupe aryle en C₆ à C₁₀ éventuellement substitué, un groupe aralkyle en C₇ à C₁₁ éventuellement substitué, un groupe hétéroaryle éventuellement substitué ou un groupe hétérocycloalkyle éventuellement substitué, à l'exception de la dihydroxyacétone, du glycéraldéhyde ainsi que des sucres en C₄ et C₅, **caractérisé en ce qu'**on fait réagir un aldéhyde de formule II
R^{a}CHO II
avec un sel de triazolium de formule IVa 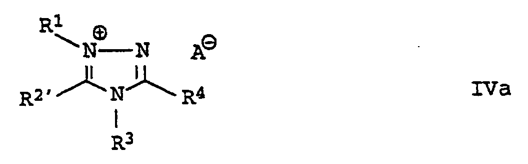 dans laquelle
R¹ et R³ sont identiques ou différents et représentent des groupes aliphatiques ayant de 1 à 30 atomes de carbone, des groupes cycloalkyle ou cycloalcényle en C₃ à C₂₀, des groupes hétérocycloalkyle ou hétérocycloalcényle en C₃ à C₂₀, des groupes alcoxy en C₂ à C₃₀ liés au noyau triazolium par l'intermédiaire d'un atome de carbone et comportant un ou plusieurs atomes d'oxygène dans la chaîne éther, des groupes halogènalkyle en C₁ à C₃₀ contenant du fluor, du chlore et/ou du brome et comportant un ou plusieurs atomes d'halogène, des groupes amino secondaires en C₂ à C₃₀ ou des groupes amino tertiaires en C₃ à C₃₀ liés au noyau triazolium par l'intermédiaire d'un atome de carbone, des groupes aryle éventuellement substitués, des groupes aralkyle éventuellement substitués et/ou des groupes hétéroaryle éventuellement substitués,
R^{2'} est le groupe R^{b}CH(OH), et dans laquelle
R⁴ est identique aux radicaux R¹ et R³ ou en est différent, ou encore représente un atome d'hydrogène, un atome d'halogène, un groupe nitro ou cyano, un groupe aliphatique ayant de 1 à 30 atomes de carbone, un groupe cycloalkyle ou cycloalcényle en C₃ à C₂₀, un groupe hétérocycloalkyle ou hétérocycloalcényle en C₃ à C₂₀, un groupe alcoxy en C₂ à C₃₀, lié au noyau triazolium par l'intermédiaire d'un atome de carbone et comportant un ou plusieurs atomes d'oxygène dans la chaîne éther, un groupe halogènalkyle en C₁ à C₃₀ contenant du fluor, du chlore et/ou du brome, et ayant un ou plusieurs atomes d'halogène, un groupe amino secondaire en C₂ à C₃₀ ou un groupe amino tertiaire en C₃ à C₃₀ lié au noyau triazolium par l'intermédiaire d'un atome de carbone, un groupe aryle éventuellement substitué, un groupe aralkyle éventuellement substitué, un groupe hétéroaryle éventuellement substitué, un groupe alcoxy -OR⁵ lié au noyau triazolium par l'intermédiaire de l'atome d'oxygène, un groupe thioéther -SR⁶ lié au noyau triazolium par l'intermédiaire de l'atome de soufre, un groupe amino -NR⁷R⁸ lié au noyau triazolium par l'intermédiaire de l'atome d'azote, un groupe acyle COR⁹ ou un groupe ester -COOR¹⁰, les radicaux R⁵, R⁶, R⁷, R⁸ et R⁹ représentant des radicaux tels ceux qui sont mentionnés pour R¹, et R¹⁰ est un groupe alkyle en C₁ à C₃₀ ou un groupe aryle ou aralkyle éventuellement substitué,
ou bien dans laquelle le radical R³, avec le radical R⁴, forme un pont alkylène ou alcénylène en C₃ à C₅ ou un pont arylène en C₆ à C₁₄, un pont aralkylène en C₇ à C₁₄ ou un pont aralcénylène en C₈ à C₁₄, et
A est l'équivalent d'un anion une ou plusieurs négativement chargé, pour assurer la neutralisation électrique de la charge du cation triazolium,
en présence d'une base auxiliaire, pour un rapport en moles sel de triazolium IVa/aldéhyde II de 1:1 à 5:1, à la condition qu'au moins l'un des radicaux R^{a} ou R^{b} soit différent d'un atome d'hydrogène.

12. Procédé selon la revendication 11, **caractérisé en ce qu'**on fait réagir les aldéhydes de formule II avec un sel de triazolium de formule IVa 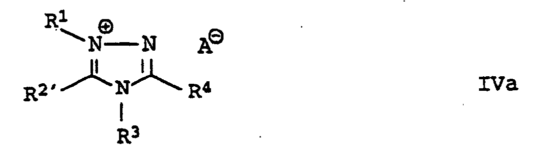 dans laquelle
R¹ et R³ sont identiques ou différents et représentent des groupes alkyle en C₁ à C₃₀, des groupes alcényle ou alcynyle en C₂ à C₃₀ ayant 1 ou 2 liaisons multiples, des groupes cycloalkyle ou cycloalcényle en C₃ à C₂₀, des groupes hétérocycloalkyle ou hétérocycloalcényle en C₃ à C₂₀, des groupes alcoxy en C₂ à C₃₀ liés au noyau triazolium par l'intermédiaire d'un atome de carbone et ayant un ou plusieurs atomes de carbone dans la chaîne éther, des groupes halogènalkyle en C₁ à C₃₀ contenant du fluor, du chlore et/ou du brome, et ayant un ou plusieurs atomes d'halogène, des groupes amino secondaires en C₂ à C₃₀ ou des groupes amino tertiaires en C₃ à C₁₀ liés au noyau triazolium par l'intermédiaire d'un atome de carbone, des groupes aryle en C₆ à C₁₄ éventuellement substitués, des groupes aralkyle en C₆ à C₁₄ éventuellement substitués, des groupes aralkyle en C₇ à C₂₀ éventuellement substitués, des groupes hétéroaryle en C₂ à C₁₅ éventuellement substitués et ayant sur le noyau de 1 à 3 atomes d'azote ou un atome d'oxygène ou un atome de soufre, ou encore ayant 1 à 2 atomes d'azote et un atome d'oxygène ou un atome de soufre,
le radical R⁴ est identique aux radicaux R¹ ou R³ ou en est différent, ou est un atome d'hydrogène, un groupe nitro ou cyano, un atome de fluor, de chlore ou de brome, un groupe alcoxy -OR⁵ lié au noyau triazolium par l'intermédiaire de l'atome d'oxygène, un groupe thioéther -SR⁶ lié au noyau triazolium par l'intermédiaire de l'atome de soufre, un groupe amino -NR⁷R⁸ lié au noyau triazolium par l'intermédiaire de l'atome d'azote, un groupe acyle COR⁹ ou un groupe ester -COOR¹⁰, les radicaux R⁵, R⁶, R⁷, R⁸ et R⁹ représentant des radicaux tels ceux qui sont mentionnés pour R¹, et R¹⁰ est un groupe alkyle en C₁ à C₃₀ ou un groupe aryle ou aralkyle éventuellement substitué,
ou bien dans laquelle le radical R⁴, avec le radical R³, forme un pont alkylène ou alcénylène en C₃ à C₅ ou un pont arylène en C₆ à C₁₄, un pont aralkylène en C₇ à C₁₄ ou un pont aralcénylène en C₈ à C₁₄, et
dans laquelle R^{2'} et A ont les significations données dans la revendication 11,
en présence d'une base auxiliaire, pour un rapport en moles sel de triazolium IVa/aldéhyde II de 1:1 à 5:1, à la condition qu'au moins l'un des radicaux R^{a} ou R^{b} soit différent d'un atome d'hydrogène.
